# EUROPEAN PATENT APPLICATION

(11) **EP 4 772 505 A1**
(43) Date of publication of application: **08.07.2026**
(21) Application number: 24860473.8
(22) Date of filing: 30.08.2024
(51) Int. Cl.: C07D 401/14, A61K 31/4545, A61K 31/496, A61K 31/501, A61P 35/00, A61P 17/10, A61P 17/14, A61P 17/02

(54) **COMPOUND FOR DEGRADING ANDROGEN RECEPTOR AND MEDICAL USE THEREOF**

(30) Priority: 01.09.2023 KR 20230116263
(71) Applicant: Ubix Therapeutics, Inc., Seoul 05836 (KR)
(72) Inventor: RYU, Je Ho, 13470 Gyeonggi-do (KR); LEE, Ji Hye, 22008 Incheon (KR); AHN, Jung Min, 21984 Incheon (KR); CHOI, Yu Ri, 21984 Incheon (KR); LEE, Song Hee, 05823 Seoul (KR); KIM, Ji Young, 13121 Gyeonggi-do (KR); WOO, Yae Jin, 13160 Gyeonggi-do (KR); LEE, Soo Hyun, 14074 Gyeonggi-do (KR); PARK, Ji Youn, 13236 Gyeonggi-do (KR); SUH, Beom Seon, 13124 Gyeonggi-do (KR); KIM, Han Wool, 15294 Gyeonggi-do (KR); KIM, So Hyuk, 05841 Seoul (KR); HONG, Hye Suk, 13607 Gyeonggi-do (KR); YUN, Whee Sahng, 05841 Seoul (KR)
(74) Representative: Graf von Stosch Patentanwaltsgesellschaft mbH
(86) International application number: PCT/KR2024/013078
(87) International publication number: WO 2025/048557

(57) **Abstract**

The present disclosure provides a compound with a specific chemical structure or a pharmaceutically-acceptable salt thereof which exhibits excellent androgen receptor (AR) degradation activity and possesses superior pharmacokinetic and pharmacodynamic properties. The present disclosure also provides a composition comprising the compound or a pharmaceutically acceptable salt thereof. Furthermore, the present disclosure provides medical uses of the compound according to the present disclosure, salt thereof, or the composition containing same for the treatment or prevention of AR-related diseases. Additionally, the present disclosure provides a method for treating or preventing an AR-related disease by administering an effective amount of the compound according to the present disclosure, salt thereof, or composition containing same to a subject in need of treatment.

## Description

### TECHNICAL FIELD

This application claims priority to Korean Patent Application No. 10-2023-0116263 filed on 1 September 2023, and all contents disclosed in the specification and drawings of the application are incorporated into this application.

The present disclosure relates to specific compound(s) having excellent androgen receptor degrading activity and excellent pharmacokinetic and pharmacodynamic properties. The present disclosure also relates to pharmaceutical compositions comprising such compounds. The present disclosure also relates to useful methods of treating androgen receptor related diseases using such compounds. That is, the present disclosure relates to the medical uses of the compounds according to the present disclosure for treating or preventing androgen receptor related diseases.

### BACKGROUND ART

Androgen hormone receptor (AR) is a transcription factor belonging to nuclear hormone receptor (NR). In the absence of androgens, AR binds to Heat Shock Protein 90 (Hsp90) in the cytosol. Hsp90 and AR are dissociated and androgen binds to AR. When AR binds to the hormone dihydrotestosterone (DHT), these complexes are translocated to the nucleus and, through a series of processes, activate the transcription of target genes.

AR contributes to the development of masculinity, but it is also a well-known oncogene in certain forms of cancer, including prostate cancer (Endocr. Rev. 2004, 25(2), 276-308). Current treatment regimens for androgen-related prostate cancer can be divided into two main categories. The first approach is to control androgen levels by removing androgens or preventing their translocation into the nucleus by interfering with the binding of its ligand, DHT. The second strategy aims to inhibit AR function by targeting AR (Nature Reviews Drug Discovery, 2013, 12, 823-824). That is, an alternative approach to treatment of prostate cancer involves deleting the AR protein. AR is an important driver of tumorigenesis in many forms of prostate cancer.

This AR can also be a major target for the treatment of acne, alopecia (especially androgenetic alopecia), cutaneous wounds, hirsutism, etc. (Arch Dermatol Res. 2012 September; 304(7): 499-510, Biomedicine & Pharmacotherapy 137 (2021) 111247), and it has been found that the expression and activation of AR also play an important role in breast cancer (especially androgen receptor-positive triple-negative breast cancer (AR+ TNBC)) (npj Breast Cancer (2020) 6:47).

Representative anti-androgen receptor drugs include enzalutamide and bicalutamide, and apalutamide has recently been approved. However, about 15-25% of prostate cancer patients do not respond to anti-androgen drugs, and the approved drug shows excellent anticancer effects in the initial stage of administration, but drug resistance develops due to continuous use, making it difficult to use them any longer. Therefore, there is an urgent need to develop new therapeutic agents.

PROTAC (Proteolysis Targeting Chimera) technology utilizes the intracellular protein degradation system (Ubiquitin-Proteasome System) to induce target protein degradation. PROTAC compounds consist of a ligand that binds to the target protein, a ligand that binds to E3 ligase proteins, and a linker connecting the two ligands. Through the formation of a target protein-PROTAC compound-E3 ligase complex, the target protein is ubiquitinated and directed to the proteasome. By degrading and removing the target protein, PROTAC technology is expected to offer superior therapeutic efficacy compared to existing inhibitors. It also overcomes the drawbacks of inhibitors, such as toxicity with high doses and resistance development with long-term administration.

Therefore, AR-degrading drugs could offer an alternative that overcomes the limitations of existing prostate cancer treatments. However, PROTAC degrading drugs are bifunctional compounds that exhibit degrading activity only when they simultaneously interact with the target protein and the E3 ligase protein. Since the degrading activity is affected by various factors such as the structure of each ligand, the structure of the linker, and the binding method with each protein, it is very difficult to find a PROTAC compound with excellent degrading activity. In addition, PROTAC degrading drugs generally have large molecular weights and low solubility, resulting in poor oral absorption. Therefore, much research is being conducted to derive PROTAC compounds that have excellent degrading activity while also having good pharmacokinetic and pharmacodynamic characteristics.

### DISCLOSURE

### Technical Problem

Therefore, the present invention aims to provide compounds exhibiting excellent androgen receptor (AR) degrading activity and exhibiting superior pharmacokinetic and pharmacodynamic characteristics, pharmaceutical compositions comprising these compounds as active ingredients, and their medical uses in the treatment or prevention of AR-related diseases.

Another challenge of the present invention is to provide a method for treating or ameliorating an AR-related disease, comprising administering a compound according to the present invention, characterized in that it degrades AR and consequently lowers AR activity, to a patient in need of such treatment, amelioration, or prevention.

### Technical Solution

### Compounds of the present invention

In order to solve the above problem, one embodiment of the present invention provides a compound represented by the following Chemical Formula 1 or a pharmaceutically acceptable salt thereof.

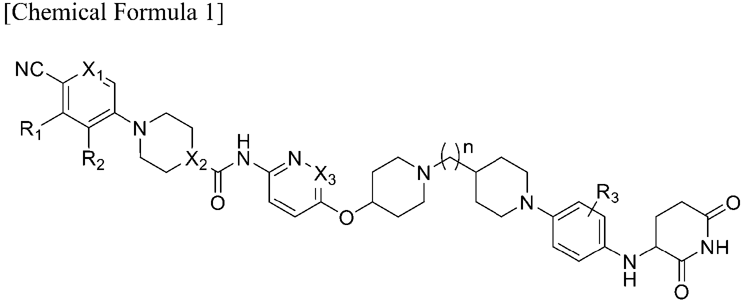

In the Chemical Formula 1,
R₁ is H, halogen (preferably, Cl), C₁₋₃alkyl, C₁₋₃alkoxy (preferably, -OCH₃), or haloC₁₋₃alkyl (preferably, -CF₃),
R₂ is H, halogen (preferably, F), or haloC₁₋₃alkyl,
X₁, X₂, and X₃ are each independently CH or N,
R₃ is H, halogen (preferably, F or Cl), C₁₋₃alkyl, or haloC₁₋₃alkyl (preferably, -CF₃), and
n is 0, 1, or 2.

The compounds of the present invention exhibit very high AR degradation activity by effectively inducing the interaction between the AR protein and the E3 ligase protein through the specific structures of the AR binding ligand of, the E3 ligase binding ligand, and the linker connecting these ligands. The compounds of the present invention exhibit significantly superior AR degradation activity and, upon administration, exhibit significantly superior pharmacological effects, such as anticancer effects. In addition, the compounds of the present invention exhibit excellent pharmacokinetic profiles, and based on this, exhibit significantly superior pharmacodynamic efficacy. Furthermore, the compounds of the present invention exhibit a very high tumor tissue-to-plasma concentration ratio upon administration, which is very advantageous in exhibiting excellent anticancer effects.

On the other hand, the compounds of the present invention have excellent degradation activity against AR mutants (T878A, M896V, F877L, H875Y, L702H, W742C, etc.). Since resistance to existing anti-androgen receptor drugs can be caused by AR mutation, the excellent AR mutant-degrading activity of the compounds of the present invention can help overcome resistance to existing drugs.

As used herein, the terms "substituent", "radical", "group", "moiety", and "fragment" may be used interchangeably.

If a substituent is described as "optionally substituted", the substituent may be (1) unsubstituted or (2) substituted with one or more of the defined substituents. If the substitutable position is unsubstituted, the default substituent is hydride radical.

As used herein, the term "alkyl" means a saturated straight chain or branched noncyclic hydrocarbon, unless the context clearly dictates otherwise, having from 1 to 10 carbon atoms. "Lower alkyl" means alkyl having from 1 to 4 carbon atoms. In one embodiment of the present invention, alkyl is a straight-chain or branched alkyl having 1 to 3 carbon atoms. Representative saturated straight-chain alkyls include -methyl, -ethyl, and -n-propyl, while saturated branched alkyls include -isopropyl.

As used herein, the term "alkoxy" means -O-(alkyl) including -OCH₃, -OCH₂CH₃, - O(CH₂)₂CH₃, and the like, wherein alkyl is as defined above.

As used herein, if the term "C₁₋₆", "C1-6", or "C1-C6" is used, it means the number of carbon atoms is from 1 to 6. For example, C₁₋₃alkyl means an alkyl which carbon number is any integer of from 1 to 3.

As used herein, the terms "halogen" and "halo" mean fluorine, chlorine, bromine or iodine. In a preferred embodiment of the present invention, the halogen is chlorine or fluorine.

As used herein, the term "haloalkyl" means an alkyl group wherein one or more hydrogen atoms are substituted with halogen atoms. For example, the haloalkyl includes - CF₃, -CHF₂, -CH₂F, -CBr₃, -CHBr₂, -CH₂Br, -CCl₃, -CHCl₂, -CH₂CI, -CI₃, -CHI₂, -CH₂I, - CH₂-CF₃, -CH₂-CHF₂, -CH₂-CH₂F, -CH₂-CBr₃, -CH₂-CHBr₂, -CH₂-CH₂Br, -CH₂-CCl₃, - CH₂-CHCl₂, -CH₂-CH₂CI, -CH₂-CI₃, -CH₂-CHI₂, -CH₂-CH₂I, and the like, wherein alkyl and halogen are as described above. In a preferred embodiment of the present invention, haloalkyl is -CF₃.

As used herein, * or 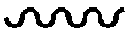 means connected to another moiety.

In various aspects such as AR degradation activity, (metabolism) stability, pharmacokinetic properties, pharmacodynamic properties, and the like, a preferred embodiment of the present invention provides a compound represented by the Chemical Formula 1 or a pharmaceutically acceptable salt thereof, wherein
R₁ is H, Cl, -CH₃, -OCH₃, or -CF₃,
R₂ is H or F,
X₁, X₂, and X₃ are each independently CH or N,
R₃ is H, F, Cl, -CH₃, or -CF₃, and
n is 0, 1, or 2.

In various aspects such as AR degradation activity, (metabolism) stability, pharmacokinetic properties, pharmacodynamic properties, and the like, a more preferred embodiment of the present invention provides a compound represented by the Chemical Formula 1 or a pharmaceutically acceptable salt thereof, wherein
R₁ is Cl, -OCH₃, or -CF₃,
R₂ is H or F,
X₁, X₂, and X₃ are each independently CH or N,
R₃ is F, Cl, or -CF₃, and
n is 0 or 1.

In various aspects such as AR degradation activity, (metabolism) stability, pharmacokinetic properties, pharmacodynamic properties, and the like, a more preferred embodiment of the present invention provides a compound represented by the Chemical Formula 1 or a pharmaceutically acceptable salt thereof, wherein
R₁ is Cl, -OCH₃, or -CF₃,
R₂ is H or F,
X₁ and X₂ are CH and X₃ is CH or N,
R₃ is F or Cl (more preferably, 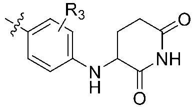 is 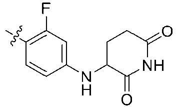 or 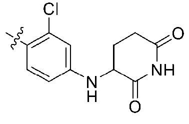 ), and
n is 1.

In particular, the structure of the E3 ligase binding ligand, for example, the position at which the linker is connected to the benzene ring, the position at which R₃ is connected to the benzene ring, etc., is important in the AR degradation activity of the compound of the present invention.

A preferred embodiment of the present disclosure is also,
1-(4-cyano-3-(trifluoromethyl)phenyl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperidine-4-carboxamide (example 1),
(S)-1-(4-cyano-3-(trifluoromethyl)phenyl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperidine-4-carboxamide (example 2),
(R)-1-(4-cyano-3-(trifluoromethyl)phenyl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperidine-4-carboxamide (example 3),
1-(4-cyano-3-(trifluoromethyl)phenyl)-N-(5-((1'-(4-((2,6-dioxopiperidin-3-yl)amino)-2-fluorophenyl)-[1,4'-bipiperidin]-4-yl)oxy)pyridin-2-yl)piperidine-4-carboxamide (example 4),
1-(6-cyano-5-(trifluoromethyl)pyridin-3-yl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperidine-4-carboxamide (example 5),
4-(4-cyano-3-(trifluoromethyl)phenyl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperazine-1-carboxamide (example 6),
1-(4-cyano-3-(trifluoromethyl)phenyl)-N-(6-((1-((1-(4-((2,6-dioxopiperidin-3-yl)amino)-2-fluorophenyl)piperidin-4-yl)methyl)piperidin-4-yl)oxy)pyridazin-3-yl)piperidine-4-carboxamide (example 7),
1-(3-chloro-4-cyanophenyl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperidine-4-carboxamide (example 8),
(S)-1-(3-chloro-4-cyanophenyl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperidine-4-carboxamide (example 9), (R)-1-(3-chloro-4-cyanophenyl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperidine-4-carboxamide (example 10),
1-(4-cyano-2-fluoro-3-(trifluoromethyl)phenyl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperidine-4-carboxamide (example 11),
1-(3-chloro-4-cyano-2-fluorophenyl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperidine-4-carboxamide (example 12),
1-(4-cyano-3-methoxyphenyl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperidine-4-carboxamide (example 13),
1-(4-cyano-2-fluoro-3-(trifluoromethyl)phenyl)-N-(6-((1-((1-(4-((2,6-dioxopiperidin-3-yl)amino)-2-fluorophenyl)piperidin-4-yl)methyl)piperidin-4-yl)oxy)pyridazin-3-yl)piperidine-4-carboxamide (example 14),
1-(3-chloro-4-cyano-2-fluorophenyl)-N-(6-((1-((1-(4-((2,6-dioxopiperidin-3-yl)amino)-2-fluorophenyl)piperidin-4-yl)methyl)piperidin-4-yl)oxy)pyridazin-3-yl)piperidine-4-carboxamide (example 15),
1-(4-cyano-3-methoxyphenyl)-N-(6-((1-((1-(4-((2,6-dioxopiperidin-3-yl)amino)-2-fluorophenyl)piperidin-4-yl)methyl)piperidin-4-yl)oxy)pyridazin-3-yl)piperidine-4-carboxamide (example 16),
N-(5-((1-((1-(2-chloro-4-((2,6-dioxopiperidin-3-yl)amino)phenyl)piperidin-4-yl)methyl)piperidin-4-yl)oxy)pyridin-2-yl)-1-(4-cyano-3-(trifluoromethyl)phenyl)piperidine-4-carboxamide (example 17), or
1-(4-cyano-3-(trifluoromethyl)phenyl)-N-(5-((1-((1-(4-((2,6-dioxopiperidin-3-yl)amino)-2-(trifluoromethyl)phenyl)piperidin-4-yl)methyl)piperidin-4-yl)oxy)pyridin-2-yl)piperidine-4-carboxamide (example 18) or a pharmaceutically acceptable salt thereof.

As used herein, the term "pharmaceutically acceptable salt(s)" refers to a salt prepared from active compounds according to the present disclosure with relatively non-toxic acids or bases, depending on the particular substituents of those compounds. When the compounds have a relatively acidic group, base-added salts can be obtained by contacting the neutral compounds with a sufficient amount of the desired base and a pure or inert solvent. Suitable pharmaceutically acceptable base addition salts include, but are not limited to sodium, potassium, calcium, aluminum, organic amino, magnesium salts and the like. When the compounds have a relatively basic group, acid-added salts can be obtained by contacting the neutral compounds with a sufficient amount of the desired acid and pure or inert solvent. Suitable pharmaceutically acceptable acid addition salts include salts derived from non-toxic organic acids including, but are not limited to, acetic acid, propionic acid, isobutyl acid, oxalic acid, maleic acid, malonic acid, benzoic acid, succinic acid, suberic acid, fumaric acid, mandelic acid, phthalic acid, benzenesulfonic acid, p-tolylsulfonic acid, citric acid, tartaric acid, methanesulfonic acid, and the like, and non-toxic inorganic acids including, but are not limited to, hydrochloric acid, hydrobromic acid, nitric acid, carbonic acid, monohydrogencarbonic acid, phosphoric acid, monohydrogenphosphoric acid, dihydrogenphosphoric acid, sulfuric acid, monohydrogensulfuric acid, hydrogen iodide, phosphorous acid and the like. Also it includes a salt of amino acid such as arginate or its analogues, and it also includes analogues of organic acid such as glucuronic or galacturonic acid. Some specific compounds of this disclosure have both basic and acidic functionality for the conversion of compounds with a basic or acidic portion (addition) salts. Other examples of salts are well known through literature known in the art to which the present invention pertains.

As used herein, the phrase "compound(s) of this/the invention" includes any compound(s) of Chemical Formula 1, as well as clathrates, hydrates, solvates, polymorphs, or isotope-labeled analogues thereof. And, even if the term "compound(s) of the invention" does not mention its pharmaceutically acceptable sat, the term includes salts thereof. In one embodiment, the compounds of this disclosure include stereo-chemically pure compounds, e.g., those substantially free (e.g., greater than 85% ee, greater than 90% ee, greater than 95% ee, greater than 97% ee, or greater than 99% ee) of other stereoisomers. That is, if the compounds of Chemical Formula 1 according to the present disclosure or salts thereof are tautomers and/or stereoisomers (e.g., geometrical isomers and conformational isomers), such isolated isomers and their mixtures also are included in the scope of this disclosure. If the compounds of the present disclosure or salts thereof have an asymmetric carbon in their structures, their active optical isomers and their racemic mixtures also are included in the scope of this disclosure.

As used herein, the term "hydrate" means a compound or its salt according to this disclosure that further includes a stoichiometric or non-stoichiometric amount of water bound by non-covalent intermolecular forces.

As used herein, the term "clathrate" means a compound or its salt in the form of a crystal lattice that contains spaces (e.g., channels) that have a guest molecule (e.g., a solvent or water) trapped within.

As used herein, the term "isotope-labeled analogue(s)" refers to a compound in which one or more atoms of a compound of the present disclosure are replaced with an atom (isotopologue) having an atomic mass or mass number different from the atomic mass or mass number normally found in nature. The compounds of the present disclosure may also contain unnatural proportions of atomic isotopes at one or more atoms constituting such compounds. Examples of isotopes that may be incorporated into the compounds described herein include isotopes of hydrogen, carbon, nitrogen, oxygen, phosphorus, sulfur, fluorine, and chlorine, such as ²H ("D"), ³H, ¹³C, ¹⁴C, ¹⁵N, ¹⁸O, ¹⁷O, ³¹P, ³²P, ³⁵S, ¹⁸F, and ³⁶Cl, respectively. For example, the compounds described herein may have one or more H atoms replaced with deuterium. Unless otherwise specified, the compounds described herein are intended to include compounds that differ only in the presence of one or more isotopically enriched atoms. For example, compounds having the structure according to the present disclosure except for the replacement of hydrogens by deuterium or tritium, or the replacement of carbons by ¹³C- or ¹⁴C-enriched carbons, are within the scope of the present disclosure. In some embodiments, certain isotope-labeled compounds, such as those labeled with ³H and ¹⁴C, may be useful for analyzing compound and/or substrate tissue distribution. Tritiated (³H) and carbon-14 (¹⁴C) isotopes may be particularly preferred due to their ease of preparation and detection. Furthermore, replacement with heavier isotopes, such as deuterium, may offer certain therapeutic advantages due to greater metabolic stability, such as increased *in vivo* half-life or reduced dosage requirements, and may therefore be desirable in some circumstances. Isotope-labeled compounds can generally be prepared by procedures similar to those disclosed herein, for example in the Examples section, by replacing the non-isotopically labeled reagent with an isotopically labeled reagent.

As used herein, the term "purified" means that when isolated, the isolate is greater than 90% pure, in one embodiment greater than 95% pure, in another embodiment greater than 99% pure and in another embodiment greater than 99.9% pure.

### Methods for preparing the compound of the present invention

One embodiment of the present invention provides a method for preparing the compound of chemical formula 1 according to the present disclosure.

The compound of chemical formula J used to synthesize the compound of the present invention can be prepared using the method of Reaction Scheme 1 or 2 below.

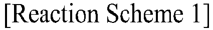

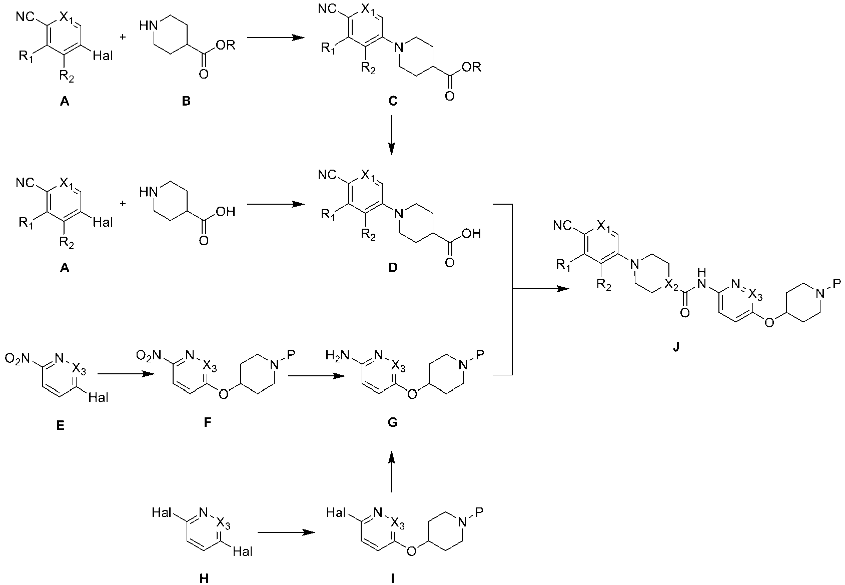

In the above reaction scheme 1, Hal is a halogen, R is an alkyl, P is a protecting group, X₂ is CH, and the remaining definitions are the same as those for chemical formula 1.

The compound of chemical formula D can be prepared by reacting the compound of chemical formula A with the compound of chemical formula B at high temperature or by a metal catalyst followed by hydrolysis, or by reacting the compound of chemical formula A with piperidine-4-carboxylic acid at high temperature.

The compound of chemical formula G can be prepared by reacting the compound of chemical formula F, which is prepared by reacting the compound of chemical formula E with piperidin-4-ol having a protected amine group, with a reduction reaction, or by reacting the compound of chemical formula I, which is prepared by reacting the compound of chemical formula H with piperidin-4-ol having a protected amine group, with an amination reaction.

The compound of chemical formula J can be prepared by reacting the compound of chemical formula D with the compound of chemical formula G at room temperature using an appropriate coupling reagent.

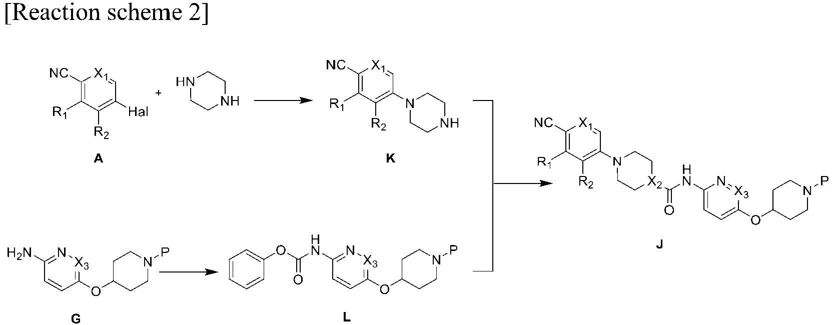

In the above reaction scheme 2, Hal is a halogen, P is a protecting group, X₂ is N, and the remaining definitions are the same as those for the preceding chemical formula 1.

The compound of chemical formula A can be reacted with piperazine at high temperature to produce a compound of chemical formula K. The compound of chemical formula G can be reacted with phenyl chloroformate at room temperature to produce a compound of chemical formula L. The compound of chemical formula K can be reacted with the compound of chemical formula L at high temperature to produce a compound of chemical formula J.

The compound of the present invention can be prepared by the method of the following reaction scheme 3 or 4.

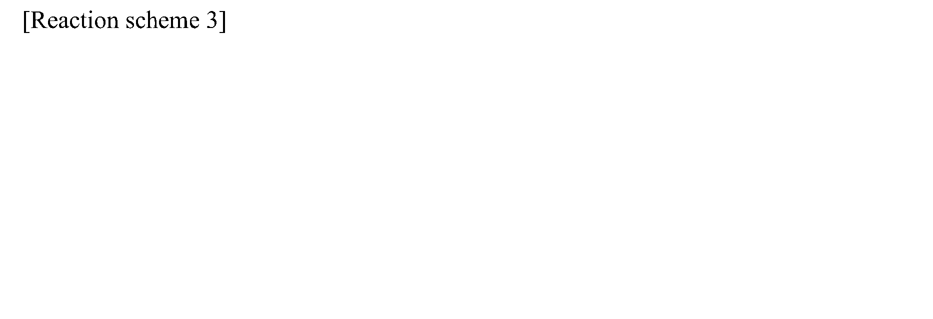

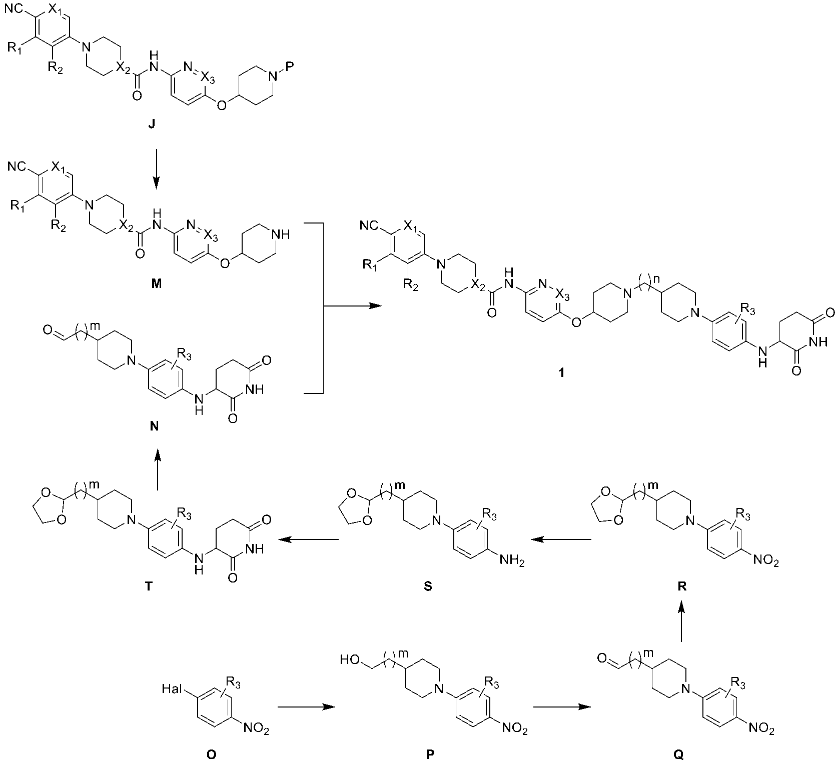

In the above reaction scheme 3, Hal is a halogen, P is a protecting group, n is 1 or 2, m is 0 or 1, and the remaining definitions are the same as those for the preceding chemical formula 1.

The compound of chemical formula P, which is prepared by reacting a compound of chemical formula O with a hydroxyalkyl-substituted piperidine, can be sequentially subjected to oxidation, acetal protection, reduction, and alkylation reactions to prepare a compound of chemical formula T. The compound of chemical formula N, which is prepared by deprotection of the compound of chemical formula T, and the compound of chemical formula M, which is prepared by deprotection of the compound of chemical formula J, can be subjected to a reductive amination reaction at room temperature to prepare a compound of chemical formula 1. The compound of chemical formula T, which has stereoselectivity, can be obtained through a stereoselective separation method such as SFC (Supercritical Fluid Chromatography).

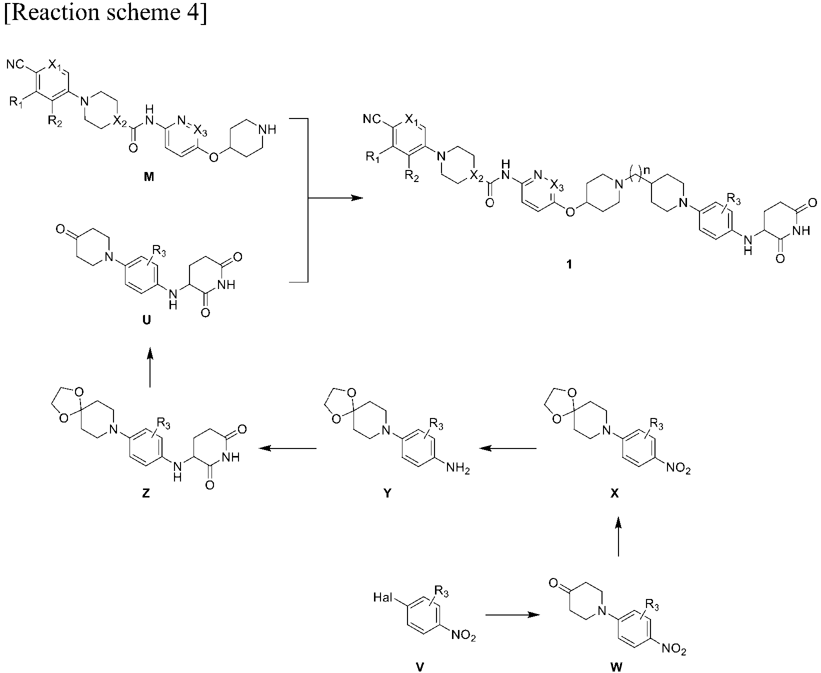

In the above reaction scheme 4, Hal is a halogen, n is 0, and the remaining definitions are the same as those for the above chemical formula 1.

The compound of chemical formula V is reacted with 4-piperidone to prepare the compound of chemical formula W, which is sequentially subjected to acetal protection, reduction, and alkylation reactions to produce the compound of chemical formula Z. The compound of chemical formula 1 can be produced by a reductive amination reaction at room temperature between the compound of chemical formula U, which is produced by deprotection of the compound of chemical formula Z, and the compound of chemical formula M. The compound of chemical formula Z, which has stereo selectivity, can be obtained through a stereoselective separation method such as SFC.

The reaction conditions for the above reactions can be modified as known in the art.

The preparation methods of the above reaction schemes 1 to 4 can be further detailed in the examples described below.

### Medical uses and methods of treatment of the compounds according to the present invention

The present invention further provides methods for treating a disease or condition in a subject having or susceptible to having such a disease or condition, by administering to the subject a therapeutically-effective amount of one or more compounds as described above. In one embodiment, the treatment is preventative treatment. In another embodiment, the treatment is palliative treatment. In another embodiment, the treatment is restorative treatment.

### 1. Diseases or conditions

The compounds of the present invention for degrading AR are useful for various therapeutic or prophylactic uses (e.g., cancer, prostate cancer, Kennedy disease). These compounds can be used to degrade AR to lower AR activity, and can also be used to treat AR-related diseases or to prevent exacerbation of these diseases. Accordingly, the present invention provides a method for degrading AR in a cell. In this method the cells are contacted with an effective amount of a compound of the invention. In one embodiment, the cell is present in a subject. The method of the present invention comprises administering to a subject in need of treatment or prevention a pharmaceutical composition comprising a therapeutically or prophylactically effective amount of a compound according to the present invention.

In one embodiment, the present invention provides a method of degrading AR in a cell of an AR-associated disease. For example, the present invention can be used to degrade AR in cells of a subject having an AR-related disease, which will be described later, and consequently lower AR activity. In another embodiment of the present invention, the present invention can be used to degrade AR in cells of cancer, in particular prostate cancer.

In another embodiment, the present invention provides a method of treating an AR-related disease, comprising administering to a subject a therapeutically effective amount of a compound of Chemical Formula 1 or a pharmaceutically acceptable salt thereof. Such a method comprises administering to a subject in need of treatment an amount of a compound of the invention sufficient to degrade AR, i.e., a therapeutically effective amount. In such a method, a compound of the present invention may be administered to the subject in the form of a pharmaceutical composition described herein.

In the present invention, AR-related diseases include, but are not limited to, asthma, multiple sclerosis, cancer (especially prostate cancer, breast cancer (especially androgen receptor positive triple negative breast cancer (AR+ TNBC))), Kennedy's disease, acne, alopecia (especially androgenetic alopecia), cutaneous wound, Hirsutism, ciliopathy, cleft palate, diabetes, heart disease, hypertension, inflammatory bowel disease, mental retardation, mood disorder, obesity, refractive error, infertility, Angelman syndrome, Canavan disease, Coeliac disease, Charcot-Marie-Tooth disease, cystic fibrosis, Duchenne muscular dystrophy, haemochromatosis, haemophilia, Klinefelter's syndrome, neurofibromatosis, phenylketonuria, polycystic kidney disease, Prader-Willi syndrome, sickle-cell disease, Tay-Sachs disease, Turner syndrome. In a preferred embodiment of the present invention, the AR-related disease is cancer, more preferably prostate cancer.

That is, the present invention provides a medical use of the compound of Chemical Formula 1 or a pharmaceutically acceptable salt thereof for treating or preventing the above diseases.

### 2. Subjects

Suitable subjects to be treated according to the present invention include mammalian subjects. Mammals according to the present disclosure include, but are not limited to, human, canine, feline, bovine, caprine, equine, ovine, porcine, rodents, lagomorphs, primates, and the like, and encompass mammals *in utero.*

In one embodiment, the suitable subject to be treated according to the present invention is human.

### 3. Administration and Dosing

The compounds of the present invention are generally administered in a therapeutically effective amount.

As used herein, "effective amount" refers to an amount of a compound of the invention sufficient to slow or minimize the progression of an AR-related disease or to provide a therapeutic benefit in the treatment or management of an AR-related disease. "Effective amount" also refers to an amount sufficient to inhibit or reduce AR activity, either *in vitro* or *in vivo.*

The compounds of the present invention can be administered by any suitable route in the form of a pharmaceutical composition adapted to such a route, and in a dose effective for the treatment intended. An effective dosage is typically in the range of about 0.001 to about 100 mg per kg body weight per day, preferably about 0.01 to about 50 mg/kg/day, in single or divided doses. Depending on age, species and disease or condition being treated, dosage levels below the lower limit of this range may be suitable. In other cases, still larger doses may be used without harmful side effects. Larger doses may also be divided into several smaller doses, for administration throughout the day.

### Pharmaceutical compositions of the compounds of the present invention

In another embodiment, the present invention provides a pharmaceutical composition comprising the compound of Chemical Formula 1 or a pharmaceutically acceptable salt thereof, and a pharmaceutically acceptable carrier or excipient. In one embodiment of the present invention, the pharmaceutical composition is used for the treatment or prevention of AR-related diseases, preferably prostate cancer, which is described above.

The term "pharmaceutically acceptable" means suitable for use as a pharmaceutical preparation, and generally considered safe for such use. The term also means that it has been officially approved by the governing body of a country for this use, or is listed in the Korean Pharmacopoeia or the United States Pharmacopoeia.

### Pharmaceutical compositions, dosage forms and administration routes

For the treatment of the diseases or conditions referred to above, the compounds described herein or pharmaceutically acceptable salts thereof can be administered as follows:

### Oral administration

The compounds of the present invention may be administered orally, including by swallowing, so that the compound enters the gastrointestinal tract, or absorbed into the blood stream directly from the mouth (e.g., buccal or sublingual administration).

Suitable compositions for oral administration include solid, liquid, gel or powder formulations, and have a dosage form such as tablet, lozenge, capsule, granule or powder.

Compositions for oral administration may optionally be enteric coated and may exhibit delayed or sustained release through the enteric coating. That is, the composition for oral administration according to the present invention may be a formulation having an immediate or modified release pattern.

Liquid formulations can include solutions, syrups and suspensions, which can be used in soft or hard capsules. Such formulations may include a pharmaceutically acceptable carrier, for example, water, ethanol, polyethylene glycol, cellulose, or an oil. The formulation may also include one or more emulsifying agents and/or suspending agents.

In a tablet dosage form, disintegrants include, but are not limited to, lactose, starch, sodium starch glycolate, crospovidone, croscarmellose sodium, maltodextrin, or mixtures thereof. Suitable lubricants, for use in a tablet, include, but are not limited to, talc, silicon dioxide, stearic acid, calcium, zinc or magnesium stearate, sodium stearyl fumarate and the like. Suitable binders, for use in a tablet, include, but are not limited to, gelatin, polyethylene glycol, sugars, gums, starch, polyvinyl pyrrolidone, hydroxypropyl cellulose, hydroxypropylmethyl cellulose and the like. Suitable diluents, for use in a tablet, include, but are not limited to, mannitol, xylitol, lactose, dextrose, sucrose, sorbitol, microcrystalline cellulose and starch.

Suitable solubilizers, for use in a tablet, include, but are not limited to, polysorbates, sodium lauryl sulfate, sodium dodecyl sulfate, propylene carbonate, diethyleneglycol monoethyl ether, dimethyl isosorbide, polyethylene glycol (natural or hydrogenated) castor oil, HCOR^{™} (Nikkol), oleyl ester, Gelucire^{™}, caprylic/caprylic acid mono/diglyceride, sorbitan fatty acid esters, and Solutol HS^{™}.

### Parenteral Administration

Compounds of the present disclosure may be administered directly into the blood stream, muscle, or internal organs. Suitable means for parenteral administration include intravenous, intra-muscular, subcutaneous intraarterial, intraperitoneal, intrathecal, intracranial, and the like. Suitable devices for parenteral administration include injectors (including needle and needle-free injectors) and infusion methods.

Compositions for parenteral administration may be formulated as immediate or modified release, including delayed or sustained release.

Most parenteral formulations are liquid compositions, and the liquid composition is an aqueous solution containing the active ingredient according to the present invention, a salt, a buffering agent, an isotonic agent, and the like.

Parenteral formulations may also be prepared in a dehydrated form (e.g., by lyophilization) or as sterile non-aqueous solutions. These formulations can be used with a suitable vehicle, such as sterile water. Solubility-enhancing agents may also be used in preparation of parenteral solutions.

### Topical Administration

Compounds of the present invention may be administered topically, to the skin or transdermally. Formulations for this topical administration can include lotions, solutions, creams, gels, hydrogels, ointments, foams, implants, patches and the like. Pharmaceutically acceptable carriers for topical administration formulations can include water, alcohol, mineral oil, glycerin, polyethylene glycol and the like. Topical administration can also be performed by electroporation, iontophoresis, phonophoresis and the like.

Compositions for topical administration may be formulated as immediate or modified release, including delayed or sustained release.

### Advantageous Effects

The present disclosure provides compounds having excellent AR degrading activity and superior pharmacokinetic and pharmacodynamic properties, pharmaceutical compositions comprising these as active ingredients, their medical uses (particularly for prostate cancer), and therapeutic methods comprising administering these to a subject in need of treatment or prevention. The compounds according to the present invention or pharmaceutically acceptable salts thereof are excellent in various aspects, including activity, (metabolic) stability, and pharmacodynamic properties.

### MODE FOR DISCLOSURE

Hereinafter, the present invention is described in considerable detail with examples to help those skilled in the art understand the present invention. However, the following examples are offered by way of illustration and are not intended to limit the scope of the invention. It is apparent that various changes may be made without departing from the spirit and scope of the invention or sacrificing all of its material advantages.

The analyses of the compounds prepared below were performed as follows. Nuclear magnetic resonance (NMR) spectroscopy was performed on a Jeol 400 MHz spectrometer, and chemical shifts were analyzed in ppm. LC-MS was performed using an ACQUITY UPLC H-Class PLUS from Waters. MPLC was performed using an Isolera instrument from Biotage and a Sfär Silica column, and chiral separation was performed using a 150 Prep-SFC instrument from Waters and a chiralcel column from Daicel. In addition, each starting material was a known compound, synthesized according to the literature or purchased commercially.

### Preparation of compounds of the present invention

Hereinafter, the synthesis process of some compounds of the present invention will be described, and the other compounds not mentioned below can be prepared by substituting starting materials, intermediates and/or reactants in a similar manner.

### Intermediate 1-1. 1-(4-cyano-3-(trifluoromethyl)phenyl)piperidine-4-carboxylic acid

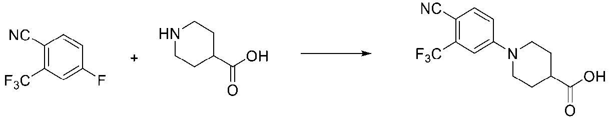

4-Fluoro-2-(trifluoromethyl)benzonitrile (5.00 g, 26.4 mmol), piperidine-4-carboxylic acid (3.41 g, 15.9 mmol), and DIPEA (11.0 ml, 79.2 mmol) were suspended in DMSO (20.0 ml) and stirred at 90°C for 16 hours. 1 M HCl aqueous solution (100 ml) was added to the reaction mixture, and extracted with EtOAc (50 ml x 3). The organic layer was dried over anhydrous sodium sulfate, filtered, and concentrated under reduced pressure. The resulting residue was recrystallized (from EtOAc) to obtain 5.98 g (76%) of an off-white solid. m/z 299.06 [M+H]⁺.

### Intermediate 1-2. 1-(6-cyano-5-(trifluoromethyl)pyridin-3-yl)piperidine-4-carboxylic acid

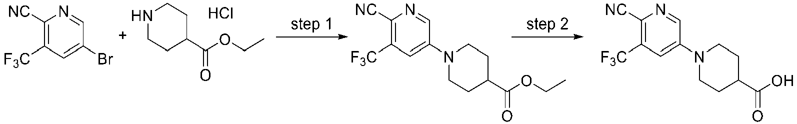

### Step 1: Synthesis of ethyl 1-(6-cyano-5-(trifluoromethyl)pyridin-3-yl)piperidine-4-carboxylate

5-Bromo-3-(trifluoromethyl)-2-pyridinecarbonitrile (500 mg, 1.99 mmol), ethyl piperidine-4-carboxylate (344 mg, 2.19 mmol), CuI (38 mg, 0.199 mmol), and K₂CO₃ (550 mg, 3.98 mmol) were suspended in DMF (3.0 ml) and stirred in a microwave at 150 °C for 1 hour. Distilled water (10 ml) was added to the reaction mixture, and extracted with EtOAc (10 ml x 2). The organic layer was dried over anhydrous sodium sulfate, filtered, and concentrated under reduced pressure. The resulting residue was purified by MPLC (30% EtOAc/Hexane) to obtain 449 mg (69%) of the title compound as a white solid. m/z 328.09 [M+H]⁺.

### Step 2: Synthesis of 1-(6-cyano-5-(trifluoromethyl)pyridin-3-yl)piperidine-4-carboxylic acid

Ethyl 1-(6-cyano-5-(trifluoromethyl)pyridin-3-yl)piperidine-4-carboxylate (449 mg, 1.37 mmol) was suspended in THF (10.0 ml) and distilled water (5.0 ml), LiOH monohydrate (230 mg, 5.49 mmol) was added, and the mixture was stirred at room temperature for 1 hour. The reaction solution was concentrated under reduced pressure, 1 M HCl aqueous solution (15 ml) was added, and the mixture was extracted with EtOAc (20 ml x 2). The organic layer was dried over anhydrous sodium sulfate, filtered, and concentrated under reduced pressure to obtain 327 mg (81%) of the title compound as a white solid. m/z 300.05 [M+H]⁺.

### Intermediate 1-3. 4-(piperazin-1-yl)-2-(trifluoromethyl)benzonitrile

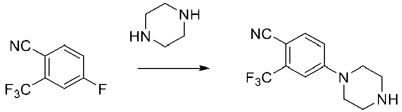

4-Fluoro-2-(trifluoromethyl)benzonitrile (1.00 g, 5.29 mmol) and piperazine (683 mg, 7.93 mmol) were suspended in DMF (10.0 ml) and stirred at 85°C for 16 hours. Distilled water (20 ml) was added to the reaction mixture, and the mixture was extracted with EtOAc (20 ml x 2). The organic layer was dried over anhydrous sodium sulfate, filtered, and concentrated under reduced pressure. The resulting residue was subjected to MPLC (10% MeOH/DCM) to obtain 868 mg (64%) of a white solid. m/z 256.13 [M+H]⁺.

### Intermediate 1-4. 1-(3-chloro-4-cyanophenyl)piperidine-4-carboxylic acid

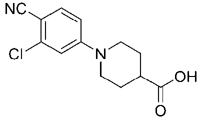

Intermediate 1-4 was synthesized in the same manner as intermediate 1-1 using 2-chloro-4-fluorobenzonitrile instead of 4-Fluoro-2-(trifluoromethyl)benzonitrile.

### Intermediate 1-5. 1-(4-cyano-2-fluoro-3-(trifluoromethyl)phenyl)piperidine-4-carboxylic acid

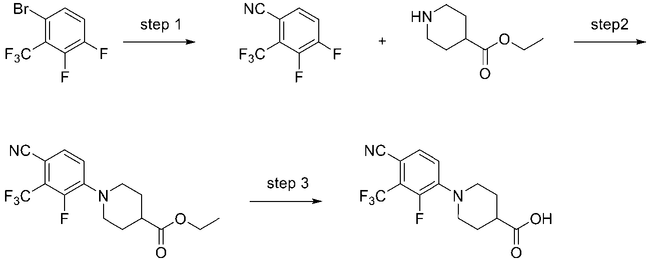

### Step 1: Synthesis of 3,4-difluoro-2-(trifluoromethyl)benzonitrile

1-Bromo-3,4-difluoro-2-(trifluoromethyl)benzene (300 mg, 1.15 mmol) and CuCN (113 mg, 1.27 mmol) were suspended in NMP (3.0 ml) and stirred at 150°C for 3 hours. Distilled water (10 ml) was added to the reaction mixture and extracted with EtOAc (10 ml x 2). The organic layer was washed with a saturated aqueous NaCl solution and a 50% aqueous NH₄Cl solution, dried over anhydrous sodium sulfate, filtered, and concentrated under reduced pressure to obtain 232 mg (mixture) of brown oil.

### Step 2: Synthesis of ethyl 1-(4-cyano-2-fluoro-3-(trifluoromethyl)phenyl)piperidine-4-carboxylate

3,4-Difluoro-2-(trifluoromethyl)benzonitrile (232 mg, mixture), ethyl piperidine-4-carboxylate (163 mg, 1.27 mmol), and DIPEA (0.40 mL, 2.30 mmol) were suspended in ACN (2.0 ml) and stirred at 90 °C for 16 hours. Distilled water (10 ml) was added to the reaction mixture, and extracted with EtOAc (10 ml x 2). The organic layer was dried over anhydrous sodium sulfate, filtered, and concentrated under reduced pressure to obtain a residue. The obtained residue was purified by MPLC (15% EtOAc/Hexane) to obtain 130 mg (33%, 2-step yield) of a white solid. m/z 345.29 [M+H]⁺.

### Step 3: Synthesis of 1-(4-cyano-2-fluoro-3-(trifluoromethyl)phenyl)piperidine-4-carboxylic acid

Ethyl 1-(4-cyano-2-fluoro-3-(trifluoromethyl)phenyl)piperidine-4-carboxylate (130 mg, 0.378 mmol) was suspended in a mixed solvent of THF (3.0 ml), MeOH (1.0 mL), and distilled water (1.0 ml), LiOH monohydrate (30 mg, 0.756 mmol) was added, and the mixture was stirred at room temperature for 1 hour. The reaction solution was concentrated under reduced pressure, 1 M HCl aqueous solution (15 ml) was added, and the mixture was extracted with EtOAc (20 ml x 2). The organic layer was dried over anhydrous sodium sulfate, filtered, and concentrated under reduced pressure to obtain 84 mg (70%) of the title compound, m/z 317.25 [M+H]⁺.

### Intermediate 1-6. 1-(3-chloro-4-cyano-2-fluorophenyl)piperidine-4-carboxylic acid

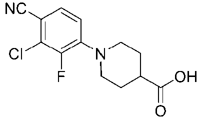

Intermediate 1-6 was synthesized using the same method as intermediate 1-1, using 2-chloro-3,4-difluorobenzonitrile instead of 4-Fluoro-2-(trifluoromethyl)benzonitrile.

### Intermediate 1-7. 1-(4-cyano-3-methoxyphenyl)piperidine-4-carboxylic acid

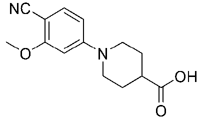

Intermediate 1-7 was synthesized in the same manner as intermediate 1-1 using 4-fluoro-2-methoxybenzonitrile instead of 4-fluoro-2-(trifluoromethyl)benzonitrile.

### Intermediate 2-1. 1-(4-((2,6-dioxopiperidin-3-yl)amino)-2-fluorophenyl)piperidine-4-carbaldehyde

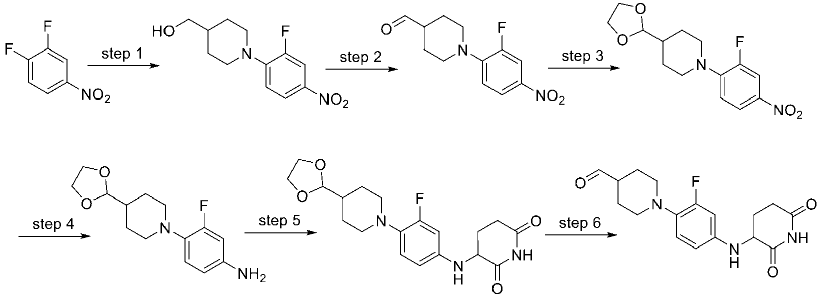

### Step 1: Synthesis of (1-(2-fluoro-4-nitrophenyl)piperidin-4-yl)methanol

1,2-Difluoro-4-nitrobenzene (10.0 g, 62.8 mmol), 4-piperidinemethanol (7.96 g, 69.1 mmol), and DIPEA (13.0 ml, 75.4 mmol) were suspended in ACN (30.0 ml) and stirred at 90 °C for 2 hours. Distilled water (100 ml) was added to the reaction mixture, and extracted with EtOAc (50 ml x 2). The organic layer was dried over anhydrous sodium sulfate, filtered, and concentrated under reduced pressure. The resulting residue was recrystallized (EtOAc/Hexane) to obtain 15.7 g (98%) of a yellow solid. m/z 255.21 [M+H]⁺.

### Step 2: Synthesis of 1-(2-fluoro-4-nitrophenyl)piperidine-4-carbaldehyde

(1-(2-Fluoro-4-nitrophenyl)piperidin-4-yl)methanol (8.00 g, 31.5 mmol) was suspended in DCM (35.0 ml), DMP (16.0 g, 37.8 mmol) was added, and the mixture was stirred at room temperature for 2 hours. Saturated aqueous Na₂S₂O₃ solution (50 ml) was added to the reaction mixture, and the mixture was extracted with DCM (50 ml x 2). The organic layer was dried over anhydrous sodium sulfate, filtered, and concentrated under reduced pressure to obtain a residue. The obtained residue was subjected to MPLC (50% EtOAc/Hexane) to obtain 5.69 g (72%) of a yellow solid. m/z 253.22 [M+H]⁺.

### Step 3: Synthesis of 4-(1,3-dioxolan-2-yl)-1-(2-fluoro-4-nitrophenyl)piperidine

1-(2-Fluoro-4-nitrophenyl)piperidine-4-carbaldehyde (5.69 g, 22.6 mmol) was suspended in toluene (100.0 ml), and pyridinium p-toluenesulfonate (568 mg, 2.26 mmol) and ethylene glycol (2.53 ml, 45.2 mmol) were added, and the mixture was stirred at 110 °C for 2 hours. Saturated NaHCO₃ aqueous solution (50 ml) was added to the reaction mixture, and extracted with DCM (50 ml x 2). The organic layer was dried over anhydrous sodium sulfate, filtered, and concentrated under reduced pressure to obtain a residue. The obtained residue was recrystallized (EtOAc/Hexane) to obtain 6.44 g (96%) of a yellow solid. m/z 297.19 [M+H]⁺.

### Step 4: Synthesis of 4-(4-(1,3-dioxolan-2-yl)piperidin-1-yl)-3-fluoroaniline

4-(1,3-Dioxolan-2-yl)-1-(2-fluoro-4-nitrophenyl)piperidine (6.44 g, 21.7 mmol) was dissolved in a mixture of EtOH (60.0 ml) and DCM (60.0 ml), Pd/C (10 wt% Pd, 665 mg) was added, and the mixture was stirred at room temperature for 24 hours under a hydrogen stream. The reaction solution was filtered and concentrated to obtain 5.36 g (93%) of an off-white solid. m/z 267.27 [M+H]⁺.

### Step 5: Synthesis of 3-((4-(4-(1,3-dioxolan-2-yl)piperidin-1-yl)-3-fluorophenyl)amino)piperidine-2,6-dione

4-(4-(1,3-Dioxolan-2-yl)piperidin-1-yl)-3-fluoroaniline (5.91 g, 22.2 mmol), 3-bromopiperidine-2,6-dione (5.96 g, 31.1 mmol), and NaHCO₃ (4.09 g, 48.8 mmol) were suspended in DMF (110.0 ml) and stirred at 60°C for 16 hours. Distilled water (200 ml) was added to the reaction mixture and extracted with EtOAc (150 ml x 3). The organic layer was dried over anhydrous sodium sulfate, filtered, and concentrated under reduced pressure to obtain a residue. The obtained residue was purified by MPLC (50% EtOAc/Hexane) to obtain 5.80 g (66%) of a green solid. m/z 378.54 [M+H]⁺.

### Step 6: Synthesis of 1-(4-((2,6-dioxopiperidin-3-yl)amino)-2-fluorophenyl)piperidine-4-carbaldehyde

3-((4-(4-(1,3-Dioxolan-2-yl)piperidin-1-yl)-3-fluorophenyl)amino)piperidine-2,6-dione (200 mg, 0.530 mmol) was suspended in THF (1.86 ml), 2 M HCl aqueous solution (1.86 ml) was added, and the mixture was stirred at 50 °C for 3 hours. The reaction solution was concentrated under reduced pressure, saturated NaHCO₃ aqueous solution (30 ml) was added, and the mixture was extracted with DCM (30 ml x 3). The organic layer was dried over anhydrous sodium sulfate, filtered, and concentrated under reduced pressure to obtain a residue. The obtained residue was recrystallized (EtOAc/Hexane) to obtain 107 mg (61%) of an off-white solid. m/z 334.23 [M+H]⁺.

### Intermediate 2-2. (S)-1-(4-((2,6-dioxopiperidin-3-yl)amino)-2-fluorophenyl)piperidine-4-carbaldehyde

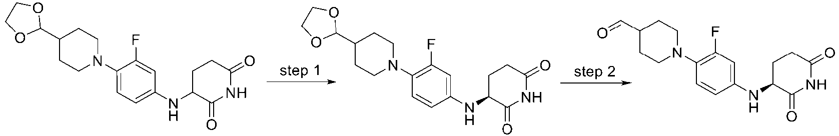

### Step 1: Synthesis of (S)-3-((4-(4-(1,3-dioxolan-2-yl)piperidin-1-yl)-3-fluorophenyl)amino)piperidine-2,6-dione

3-((4-(4-(1,3-Dioxolan-2-yl)piperidin-1-yl)-3-fluorophenyl)amino)piperidine-2,6-dione (Intermediate 2-1 Step 5, 8.60 g, 22.8 mmol) was separated via SFC (Waters 150 Prep-SFC, Daicel chiralcel IG column, mobile phase: A for CO₂, B for IPA and ACN, 65% phase B gradient, flow rate: 100 mL/min) to obtain 3.89 g (45.2%) of a green solid.

Analytical SFC (SHIMADZU LC-30AD SF, IG 50 x 4.6 mm I.D., 3.0µm particle size, mobile phase: A for CO₂, B for 0.05% DEA in IPA and ACN, 60% gradient phase B, flow rate: 3 mL/min): retention time 0.661 min, >99% ee.

### Step 2: Synthesis of (S)-1-(4-((2,6-dioxopiperidin-3-yl)amino)-2-fluorophenyl)piperidine-4-carbaldehyde

(S)-3-((4-(4-(1,3-Dioxolan-2-yl)piperidin-1-yl)-3-fluorophenyl)amino)piperidine-2,6-dione (3.00 g, 7.95 mmol) was suspended in THF (60.0 ml), 2 M HCl aqueous solution (60.0 ml) was added, and the mixture was stirred at 50 °C for 2 hours. The reaction solution was concentrated under reduced pressure, saturated NaHCO₃ aqueous solution (350 ml) was added, and the mixture was extracted with DCM (150 ml x 4). The organic layer was dried over anhydrous sodium sulfate, filtered, and concentrated under reduced pressure to obtain a residue. The obtained residue was recrystallized (EtOAc/Hexane) to obtain 2.40 g (89%) of an off-white solid. m/z 334.26 [M+H]⁺.

### Intermediate 2-3. (R)-1-(4-((2,6-dioxopiperidin-3-yl)amino)-2-fluorophenyl)piperidine-4-carbaldehyde

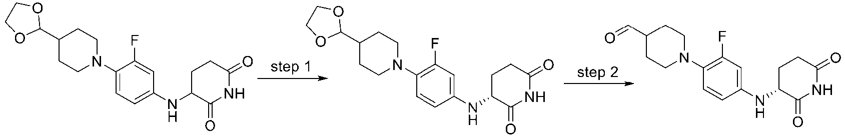

### Step 1: Synthesis of (R)-3-((4-(4-(1,3-dioxolan-2-yl)piperidin-1-yl)-3-fluorophenyl)amino)piperidine-2,6-dione

3-((4-(4-(1,3-Dioxolan-2-yl)piperidin-1-yl)-3-fluorophenyl)amino)piperidine-2,6-dione (Intermediate 2-1 Step 5, 8.60 g, 22.8 mmol) was separated via SFC (Waters 150 Prep-SFC, Daicel chiralcel IG column, mobile phase: A for CO₂, B for IPA and ACN, 65% phase B gradient, flow rate: 100 mL/min) to obtain 3.94 g (45.8%) of a green solid.

Analytical SFC (SHIMADZU LC-30AD SF, IG 50 x 4.6 mm I.D., 3.0µm particle size, mobile phase: A for CO₂, B for 0.05% DEA in IPA and ACN, 60% gradient phase B, flow rate: 3 mL/min): retention time 1.101 min, >99% ee.

### Step 2: Synthesis of (R)-1-(4-((2,6-dioxopiperidin-3-yl)amino)-2-fluorophenyl)piperidine-4-carbaldehyde

(R)-3-((4-(4-(1,3-Dioxolan-2-yl)piperidin-1-yl)-3-fluorophenyl)amino)piperidine-2,6-dione (250 mg, 0.662 mmol) was suspended in THF (2.32 ml), 2 M HCl aqueous solution (2.32 ml) was added, and the mixture was stirred at 50 °C for 1 hour. The reaction solution was concentrated under reduced pressure, saturated NaHCO₃ aqueous solution (60 ml) was added, and the mixture was extracted with DCM (20 ml x 3). The organic layer was dried over anhydrous sodium sulfate, filtered, and concentrated under reduced pressure to obtain a residue. The obtained residue was recrystallized (EtOAc/Hexane) to obtain 172 mg (78%) of an off-white solid. m/z 334.32 [M+H]⁺.

### Intermediate 2-4. 3-((3-fluoro-4-(4-oxopiperidin-1-yl)phenyl)amino)piperidine-2,6-dione

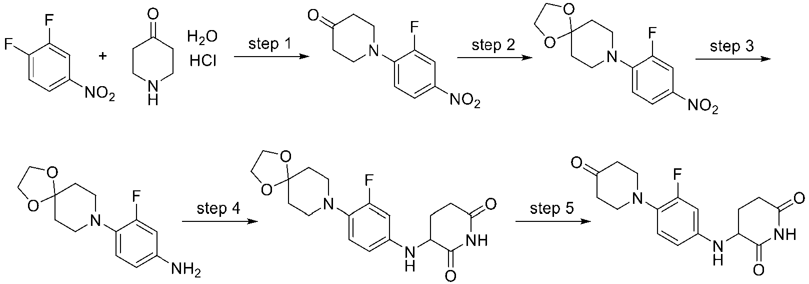

### Step 1: Synthesis of 1-(2-fluoro-4-nitrophenyl)piperidin-4-one

1,2-Difluoro-4-nitrobenzene (300 mg, 1.89 mmol), 4-piperidone monohydrate hydrochloride (348 mg, 1.95 mmol), and DIPEA (0.66 ml, 3.78 mmol) were suspended in DMF (4.0 ml) and stirred at 90 °C for 2 hours. Distilled water (20 ml) was added to the reaction mixture and extracted with EtOAc (20 ml x 2). The organic layer was dried over anhydrous sodium sulfate, filtered, and concentrated under reduced pressure to obtain a residue. The obtained residue was purified by MPLC (80% EtOAc/Hexane) to obtain 390 mg (87%) of a yellow solid. m/z 239.22 [M+H]⁺.

### Step 2: Synthesis of 8-(2-fluoro-4-nitrophenyl)-1,4-dioxa-8-azaspiro[4.5]decane

1-(2-Fluoro-4-nitrophenyl)piperidin-4-one (150 mg, 0.630 mmol) was suspended in toluene (2.0 ml), and pyridinium p-toluenesulfonate (16 mg, 0.063 mmol) and ethylene glycol (0.07 ml, 0.84 mmol) were added, and the mixture was stirred at 110 °C for 2 hours. Saturated NaHCO₃ aqueous solution (20 ml) was added to the reaction mixture, and extracted with DCM (20 ml x 2). The organic layer was dried over anhydrous sodium sulfate, filtered, and concentrated under reduced pressure to obtain a residue. The obtained residue was recrystallized (EtOAc) to obtain 147 mg (83%) of the title yellow solid. m/z 283.16 [M+H]⁺.

### Step 3: Synthesis of 3-fluoro-4-(1,4-dioxa-8-azaspiro[4.5]decan-8-yl)aniline

8-(2-Fluoro-4-nitrophenyl)-1,4-dioxa-8-azaspiro[4.5]decane (136 mg, 0.481 mmol) was dissolved in a mixture of DCM (1.0 ml) and MeOH (3.0 ml), Pd/C (10 wt% Pd, 15 mg) was added, and the mixture was stirred at room temperature for 16 hours under a stream of hydrogen. The reaction solution was filtered and concentrated to obtain 120 mg (99%) of a pink solid. m/z 253.26 [M+H]⁺.

### Step 4: Synthesis of 3-((3-fluoro-4-(1,4-dioxa-8-azaspiro[4.5]decan-8-yl)phenyl)amino)piperidine-2,6-dione

3-Fluoro-4-(1,4-dioxa-8-azaspiro[4.5]decan-8-yl)aniline (60 mg, 0.238 mmol), 3-bromopiperidine-2,6-dione (68 mg, 0.357 mmol), and NaHCO₃ (44 mg, 0.52 mmol) were suspended in DMF (1.5 ml) and stirred at 80 °C for 12 hours. Distilled water (15 ml) was added to the reaction mixture and extracted with EtOAc (15 ml x 2). The organic layer was dried over anhydrous sodium sulfate, filtered, and concentrated under reduced pressure. The resulting residue was purified by MPLC (50% EtOAc/Hexane) to give 54 mg (62%) of a green solid. m/z 364.24 [M+H]⁺.

### Step 5: Synthesis of 3-((3-fluoro-4-(4-oxopiperidin-1-yl)phenyl)amino)piperidine-2,6-dione

3-((3-Fluoro-4-(1,4-dioxa-8-azaspiro[4.5]decan-8-yl)phenyl)amino)piperidine-2,6-dione (54 mg, 0.15 mmol) was suspended in THF (0.75 ml), 2 M HCl aqueous solution (0.75 ml, 1.5 mmol) was added, and the mixture was stirred at 60 °C for 16 hours. The reaction solution was concentrated under reduced pressure, and saturated NaHCO₃ aqueous solution (20 ml) was added, followed by extraction with DCM (20 ml x 2). The organic layer was dried over anhydrous sodium sulfate, filtered, and concentrated under reduced pressure to obtain 35 mg (73%) of a blue solid. m/z 320.21 [M+H]⁺.

### Intermediate 2-5. 1-(2-chloro-4-((2,6-dioxopiperidin-3-yl)amino)phenyl)piperidine-4-carbaldehyde

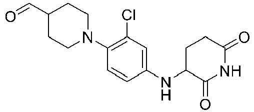

Intermediate 2-5 was synthesized in the same manner as intermediate 2-1 using 2-chloro-1-fluoro-4-nitrobenzene instead of 1,2-difluoro-4-nitrobenzene.

### Intermediate 2-6. 1-(4-((2,6-dioxopiperidin-3-yl)amino)-2-(trifluoromethyl)phenyl)piperidine-4-carbaldehyde

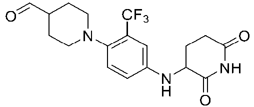

Intermediate 2-6 was synthesized in the same manner as intermediate 2-1 using 1-fluoro-4-nitro-2-(trifluoromethyl)benzene instead of 1,2-difluoro-4-nitrobenzene.

### Example 1. 1-(4-cyano-3-(trifluoromethyl)phenyl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperidine-4-carboxamide

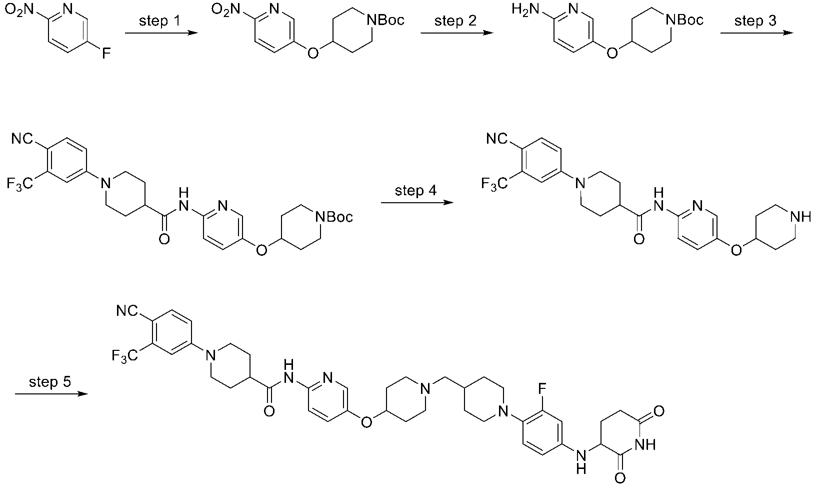

### Step 1: Synthesis of tert-butyl 4-((6-nitropyridin-3-yl)oxy)piperidine-1-carboxylate

1-(Tert-butoxycarbonyl)-4-hydroxypiperidine (8.50 g, 42.2 mmol) was suspended in THF (180 ml), 60% NaH (1.69 g, 42.2 mmol) was added at 0 °C, and the mixture was stirred at room temperature for 30 minutes. 5-Fluoro-2-nitropyridine (5.00 g, 35.2 mmol) was added to the reaction mixture, and the mixture was stirred at room temperature for 16 hours. Distilled water (200 ml) was added to the reaction mixture, and EtOAc (150 ml x 2) was extracted. The organic layer was dried over anhydrous sodium sulfate, filtered, and concentrated under reduced pressure. The resulting residue was recrystallized (EtOAc) to obtain 7.20 g (63%) of an off-white solid. m/z 324.15 [M+H]⁺.

### Step 2: Synthesis of tert-butyl 4-((6-aminopyridin-3-yl)oxy)piperidine-1-carboxylate

Tert-butyl 4-((6-nitropyridin-3-yl)oxy)piperidine-1-carboxylate (7.20 g, 22.3 mmol) was dissolved in a mixture of DCM (50 ml) and MeOH (60 ml), Pd/C (10 wt% Pd, 25 mg) was added, and the mixture was stirred at room temperature for 2 hours under a stream of hydrogen. The reaction solution was filtered and concentrated to obtain a black solid (6.54 g, quant.). m/z 294.20 [M+H]⁺.

### Step 3: Synthesis of tert-butyl 4-((6-(1-(4-cyano-3-(trifluoromethyl)phenyl)piperidine-4-carboxamido)pyridin-3-yl)oxy)piperidine-1-carboxylate

Tert-butyl 4-((6-aminopyridin-3-yl)oxy)piperidine-1-carboxylate (6.54 g, 22.3 mmol), 1-(4-cyano-3-(trifluoromethyl)phenyl)piperidine-4-carboxylic acid (intermediate 1-1, 6.65 g, 22.3 mmol), HATU (10.2 g, 26.8 mmol), and DIPEA (7.77 ml, 44.6 mmol) were suspended in DMF (45.0 ml) and stirred at room temperature for 16 hours. Distilled water (15 ml) was added to the reaction mixture and extracted with EtOAc (150 ml x 2). The organic layer was dried over anhydrous sodium sulfate, filtered, and concentrated under reduced pressure. The resulting residue was recrystallized (EtOAc) to obtain 9.23 g (72%) of an off-white solid. m/z 574.30 [M+H]⁺.

### Step 4: Synthesis of 1-(4-cyano-3-(trifluoromethyl)phenyl)-N-(5-(piperidin-4-yloxy)pyridin-2-yl)piperidine-4-carboxamide

Tert-butyl 4-((6-(1-(4-cyano-3-(trifluoromethyl)phenyl)piperidine-4-carboxamido)pyridin-3-yl)oxy)piperidine-1-carboxylate (9.23 g, 16.1 mmol) was suspended in DCM (41.0 ml), TFA (16.4 ml, 214.0 mmol) was added, and the mixture was stirred at room temperature for 4 hours. The reaction solution was concentrated under reduced pressure, and 2 M NaOH aqueous solution (150 ml) was added, followed by extraction with DCM (100 ml x 2). The organic layer was dried over anhydrous sodium sulfate, filtered, and concentrated under reduced pressure to obtain 7.52 g (99%) of an off-white solid. m/z 474.22 [M+H]⁺.

### Step 5: Synthesis of 1-(4-cyano-3-(trifluoromethyl)phenyl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperidine-4-carboxamide

1-(4-Cyano-3-(trifluoromethyl)phenyl)-N-(5-(piperidin-4-yloxy)pyridin-2-yl)piperidine-4-carboxamide (3.41 g, 7.20 mmol) and 1-(4-((2,6-dioxopiperidin-3-yl)amino)-2-fluorophenyl)piperidine-4-carbaldehyde (intermediate 2-1, 2.40 g, 7.20 mmol) were suspended in a mixture of DCM (50.0 ml) and MeOH (5.0 ml), sodium triacetoxyborohydride (3.82 g, 18.0 mmol) was added, and the mixture was stirred at room temperature for 16 hours. A saturated aqueous solution of NaHCO₃ (70 ml) was added to the reaction mixture, and the mixture was extracted with DCM (70 ml x 3). The organic layer was dried over anhydrous sodium sulfate, filtered, and concentrated under reduced pressure. The resulting residue was subjected to MPLC (10% MeOH/DCM) to obtain 4.56 g (80%) of a light-colored solid.

### Example 2. (S)-1-(4-cyano-3-(trifluoromethyl)phenyl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperidine-4-carboxamide

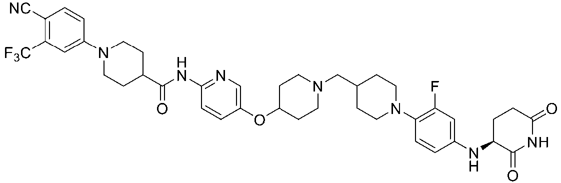

Example 2 was synthesized in the same manner as Example 1, using (S)-1-(4-((2,6-dioxopiperidin-3-yl)amino)-2-fluorophenyl)piperidine-4-carbaldehyde (Intermediate 2-2) instead of 1-(4-((2,6-Dioxopiperidin-3-yl)amino)-2-fluorophenyl)piperidine-4-carbaldehyde (Intermediate 2-1).

### Example 3. (R)-1-(4-cyano-3-(trifluoromethyl)phenyl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperidine-4-carboxamide

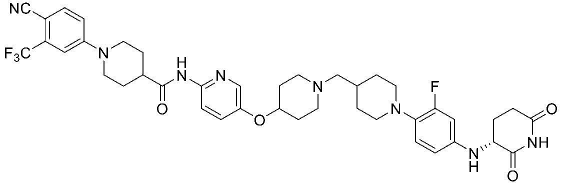

Example 3 was synthesized in the same manner as Example 1, using (R)-1-(4-((2,6-dioxopiperidin-3-yl)amino)-2-fluorophenyl)piperidine-4-carbaldehyde (Intermediate 2-3) instead of 1-(4-((2,6-Dioxopiperidin-3-yl)amino)-2-fluorophenyl)piperidine-4-carbaldehyde (Intermediate 2-1).

### Example 4. 1-(4-cyano-3-(trifluoromethyl)phenyl)-N-(5-((1'-(4-((2,6-dioxopiperidin-3-yl)amino)-2-fluorophenyl)-[1,4'-bipiperidin]-4-yl)oxy)pyridin-2-yl)piperidine-4-carboxamide

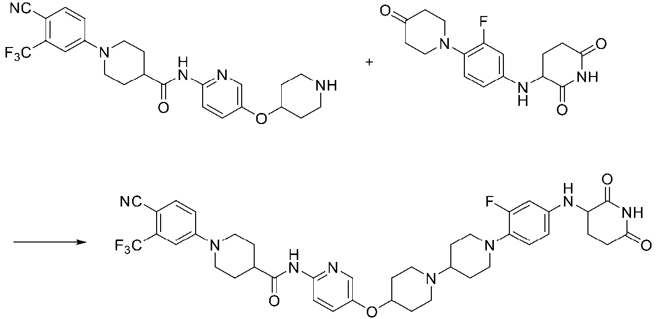

1-(4-Cyano-3-(trifluoromethyl)phenyl)-N-(5-(piperidin-4-yloxy)pyridin-2-yl)piperidine-4-carboxamide (Example 1 Step 4, 43 mg, 0.091 mmol) and 1-(4-((2,6-dioxopiperidin-3-yl)amino)-2-fluorophenyl)piperidine-4-carbaldehyde (Intermediate 2-4, 35 mg, 0.11 mmol) were suspended in a mixture of MeOH (1.0 ml) and AcOH (0.1 ml), and 2-methylpyridine borane complex (29 mg, 0.27 mmol) was added and stirred at room temperature for 16 hours. 1 M KOH aqueous solution (1.0 ml) was added to the reaction mixture and extracted with DCM (10 ml x 2). The organic layer was dried over anhydrous sodium sulfate, filtered, and concentrated under reduced pressure. The resulting residue was subjected to MPLC (10% MeOH/DCM) to obtain 13 mg (18%) of a sky-blue solid.

### Example 5. 1-(6-cyano-5-(trifluoromethyl)pyridin-3-yl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperidine-4-carboxamide

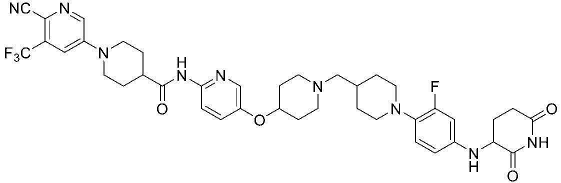

Example 5 was synthesized in the same manner as Example 1, using 1-(6-cyano-5-(trifluoromethyl)pyridin-3-yl)piperidine-4-carboxylic acid (Intermediate 1-2) instead of 1-(4-Cyano-3-(trifluoromethyl)phenyl)piperidine-4-carboxylic acid (Intermediate 1-1).

### Example 6. 4-(4-cyano-3-(trifluoromethyl)phenyl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperazine-1-carboxamide

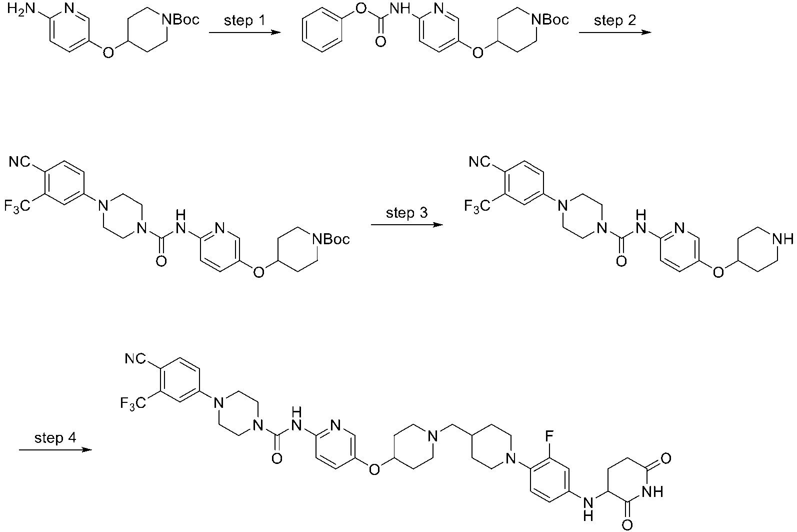

### Step 1: Synthesis of tert-butyl 4-((6-((phenoxycarbonyl)amino)pyridin-3-yl)oxy)piperidine-1-carboxylate

Tert-butyl 4-((6-aminopyridin-3-yl)oxy)piperidine-1-carboxylate (Example 1 Step 2, 100 mg, 0.341 mmol) was suspended in THF (2.0 ml), and phenyl chloroformate (0.05 mg, 0.375 mmol) and pyridine (0.03 ml, 0.375 mmol) were added at 0 °C and stirred at room temperature for 2 hours. Distilled water (15 ml) was added to the reaction solution, and the precipitated solid was filtered and dried under vacuum to obtain 105 mg (74%) of a white solid. m/z 414.16 [M+H]⁺.

### Step 2: Synthesis of tert-butyl 4-((6-(4-(4-cyano-3-(trifluoromethyl)phenyl)piperazine-1-carboxamido)pyridin-3-yl)oxy)piperidine-1-carboxylate

Tert-butyl 4-((6-((phenoxycarbonyl)amino)pyridin-3-yl)oxy)piperidine-1-carboxylate (105 mg, 0.254 mmol), 4-(piperazin-1-yl)-2-(trifluoromethyl)benzonitrile (intermediate 1-3, 65 mg, 0.254 mmol), and DMAP (30 mg, 0.127 mmol) were suspended in DMF (1.0 ml) and stirred at 80 °C for 3 hours. Distilled water (15 ml) was added to the reaction mixture and extracted with EtOAc (25 ml x 2). The organic layer was dried over anhydrous sodium sulfate, filtered, and concentrated under reduced pressure. The resulting residue was purified by MPLC (10% MeOH/DCM) to obtain 126 mg (86%) of a white solid. m/z 575.29 [M+H]⁺.

### Step 3: Synthesis of 4-(4-cyano-3-(trifluoromethyl)phenyl)-N-(5-(piperidin-4-yloxy)pyridin-2-yl)piperazine-1-carboxamide

Tert-butyl 4-((6-(4-(4-cyano-3-(trifluoromethyl)phenyl)piperazine-1-carboxamido)pyridin-3-yl)oxy)piperidine-1-carboxylate (126 mg, 0.219 mmol) was suspended in DCM (1.0 ml), 4 M HCl in dioxane (0.27 ml, 1.10 mmol) was added, and the mixture was stirred at room temperature for 2 hours. The reaction solution was concentrated under reduced pressure, saturated aqueous NaHCO₃ solution (15 ml) was added, and the mixture was extracted with DCM (25 ml x 2). The organic layer was dried over anhydrous sodium sulfate, filtered, and concentrated under reduced pressure to obtain 100 mg (82%) of the title compound as a white solid. m/z 475.24 [M+H]⁺.

### Step 4: Synthesis of 4-(4-cyano-3-(trifluoromethyl)phenyl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperazine-1-carboxamide

4-(4-Cyano-3-(trifluoromethyl)phenyl)-N-(5-(piperidin-4-yloxy)pyridin-2-yl)piperazine-1-carboxamide (20 mg, 0.042 mmol) and 1-(4-((2,6-dioxopiperidin-3-yl)amino)-2-fluorophenyl)piperidine-4-carbaldehyde (intermediate 2-1, 14 mg, 0.042 mmol) were suspended in a mixture of DCM (1.0 ml) and MeOH (0.25 ml), sodium triacetoxyborohydride (22 mg, 0.11 mmol) was added, and the mixture was stirred at room temperature for 16 hours. A saturated aqueous solution of NaHCO₃ (15 ml) was added to the reaction mixture, and the mixture was extracted with DCM (15 ml x 2). The organic layer was dried over anhydrous sodium sulfate, filtered, and concentrated under reduced pressure. The resulting residue was subjected to MPLC (10% MeOH/DCM) to obtain 16 mg (48%) of a light-colored solid.

### Example 7. 1-(4-cyano-3-(trifluoromethyl)phenyl)-N-(6-((1-((1-(4-((2,6-dioxopiperidin-3-yl)amino)-2-fluorophenyl)piperidin-4-yl)methyl)piperidin-4-yl)oxy)pyridazin-3-yl)piperidine-4-carboxamide

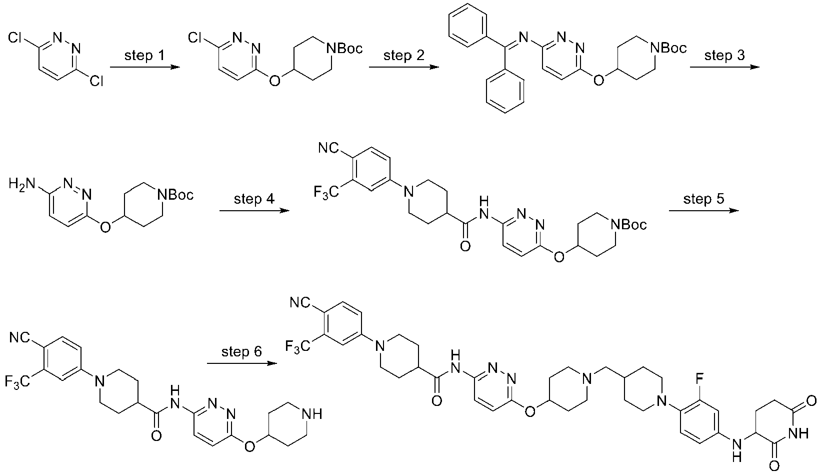

### Step 1: Synthesis of tert-butyl 4-((6-chloropyridazin-3-yl)oxy)piperidine-1-carboxylate

1-(Tert-butoxycarbonyl)-4-hydroxypiperidine (7.08 g, 35.2 mmol) was suspended in THF (350 ml), 60% NaH (2.11 g, 52.8 mmol) was added at 0 °C, and the mixture was stirred at room temperature for 1 hour. 3,6-dichloropyridazine (5.00 g, 35.2 mmol) was added to the reaction mixture, and the mixture was stirred at room temperature for 16 hours. Distilled water (300 ml) was added to the reaction mixture, and EtOAc (150 ml x 2) was extracted. The organic layer was dried over anhydrous sodium sulfate, filtered, and concentrated under reduced pressure. The resulting residue was recrystallized (EtOAc) to obtain 8.42 g (76%) of a white solid. m/z 336.36 [M+Na]⁺.

### Step 2: Synthesis of tert-butyl 4-((6-((diphenylmethylene)amino)pyridazin-3-yl)oxy)piperidine-1-carboxylate

Tert-butyl 4-((6-chloropyridazin-3-yl)oxy)piperidine-1-carboxylate (8.42 g, 26.8 mmol), benzophenone imine (5.35 g, 29.5 mmol), and Cs₂CO₃ (26.2 g, 80.4 mmol) were suspended in toluene (150.0 ml), Pd₂(dba)₃ (1.54 g, 2.68 mmol) and BINAP (3.34 g, 5.36 mmol) were added, and the mixture was stirred at 100 °C for 16 hours. The reaction solution was filtered and concentrated, and the resulting residue was subjected to MPLC (30% EtOAc/Hexane) to obtain 9.42 g (76%) of a white solid. m/z 459.31 [M+H]⁺.

### Step 3: Synthesis of tert-butyl 4-((6-aminopyridazin-3-yl)oxy)piperidine-1-carboxylate

Tert-butyl 4-((6-((diphenylmethylene)amino)pyridazin-3-yl)oxy)piperidine-1-carboxylate (9.42 g, 20.5 mmol) was dissolved in MeOH (100.0 ml), and hydroxylamine hydrochloride (1.57 g, 22.6 mmol) and NaOAc (4.20 g, 51.3 mmol) were added. The mixture was stirred at room temperature for 3 hours. The reaction solution was concentrated under reduced pressure, and the residue obtained was subjected to MPLC (10% MeOH/DCM) to obtain 4.66 g (77%) of a white solid. m/z 295.23 [M+H]⁺.

### Step 4: Synthesis of tert-butyl 4-((6-(1-(4-cyano-3-(trifluoromethyl)phenyl)piperidine-4-carboxamido)pyridazin-3-yl)oxy)piperidine-1-carboxylate

Tert-butyl 4-((6-aminopyridazin-3-yl)oxy)piperidine-1-carboxylate (14.3 g, 48.6 mmol), 1-(4-cyano-3-(trifluoromethyl)phenyl)piperidine-4-carboxylic acid (intermediate 1-1, 14.5 g, 48.6 mmol), HATU (22.2 g, 58.3 mmol), and DIPEA (16.9 ml, 97.2 mmol) were suspended in DMF (100.0 ml) and stirred at room temperature for 16 hours. Distilled water (150 ml) was added to the reaction mixture and extracted with EtOAc (200 ml x 2). The organic layer was dried over anhydrous sodium sulfate, filtered, and concentrated under reduced pressure to obtain a residue. The obtained residue was recrystallized (EtOAc) to obtain 23.3 g (83%) of an off-white solid. m/z 597.28 [M+Na]⁺.

### Step 5: Synthesis of 1-(4-cyano-3-(trifluoromethyl)phenyl)-N-(6-(piperidin-4-yloxy)pyridazin-3-yl)piperidine-4-carboxamide

Tert-butyl 4-((6-(1-(4-cyano-3-(trifluoromethyl)phenyl)piperidine-4-carboxamido)pyridazin-3-yl)oxy)piperidine-1-carboxylate (21.0 g, 36.5 mmol) was suspended in DCM (280.0 ml), TFA (42.0 ml, 548 mmol) was added, and the mixture was stirred at room temperature for 20 hours. The reaction solution was concentrated under reduced pressure, and saturated 2M NaOH aqueous solution (150 ml) was added, followed by extraction with DCM (200 ml x 2). The organic layer was dried over anhydrous sodium sulfate, filtered, and concentrated under reduced pressure to obtain 16.8 g (97%) of an off-white solid. m/z 475.23 [M+H]⁺.

### Step 6: Synthesis of 1-(4-cyano-3-(trifluoromethyl)phenyl)-N-(6-((1-((1-(4-((2,6-dioxopiperidin-3-yl)amino)-2-fluorophenyl)piperidin-4-yl)methyl)piperidin-4-yl)oxy)pyridazin-3-yl)piperidine-4-carboxamide

1-(4-Cyano-3-(trifluoromethyl)phenyl)-N-(6-(piperidin-4-yloxy)pyridazin-3-yl)piperidine-4-carboxamide (30 mg, 0.0063 mmol) and 1-(4-((2,6-dioxopiperidin-3-yl)amino)-2-fluorophenyl)piperidine-4-carbaldehyde (intermediate 2-1, 21 mg, 0.063 mmol) were suspended in a mixture of DCM (1.0 ml) and MeOH (0.25 ml), sodium triacetoxyborohydride (33 mg, 0.16 mmol) was added, and the mixture was stirred at room temperature for 16 hours. A saturated aqueous solution of NaHCO₃ (15 ml) was added to the reaction mixture, and the mixture was extracted with DCM (15 ml x 2). The organic layer was dried over anhydrous sodium sulfate, filtered, and concentrated under reduced pressure. The resulting residue was subjected to MPLC (10% MeOH/DCM) to obtain 17 mg (34%) of an off-white solid.

### Example 8. 1-(3-chloro-4-cyanophenyl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperidine-4-carboxamide

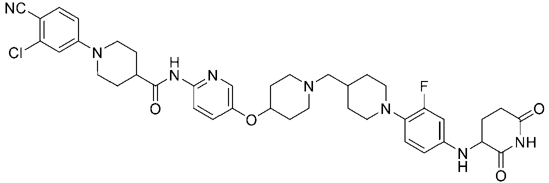

Example 8 was synthesized in the same manner as Example 1, using 1-(3-chloro-4-cyanophenyl)piperidine-4-carboxylic acid (intermediate 1-4) instead of 1-(4-cyano-3-(trifluoromethyl)phenyl)piperidine-4-carboxylic acid (intermediate 1-1).

### Example 9. (S)-1-(3-chloro-4-cyanophenyl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperidine-4-carboxamide

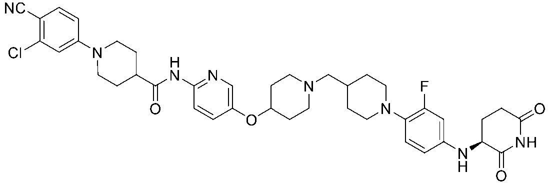

Example 9 was synthesized in the same manner as Example 1, using 1-(3-chloro-4-cyanophenyl)piperidine-4-carboxylic acid (intermediate 1-4) and (S)-1-(4-((2,6-dioxopiperidin-3-yl)amino)-2-fluorophenyl)piperidine-4-carbaldehyde (intermediate 2-2) instead of 1-(4-cyano-3-(trifluoromethyl)phenyl)piperidine-4-carboxylic acid (intermediate 1-1) and 1-(4-((2,6-dioxopiperidin-3-yl)amino)-2-fluorophenyl)piperidine-4-carbaldehyde (intermediate 2-1), respectively.

### Example 10. (R)-1-(3-chloro-4-cyanophenyl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperidine-4-carboxamide

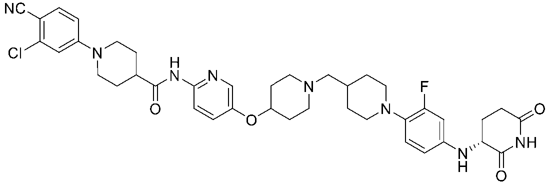

Example 10 was synthesized in the same manner as Example 1, using 1-(3-chloro-4-cyanophenyl)piperidine-4-carboxylic acid (intermediate 1-4) and (R)-1-(4-((2,6-dioxopiperidin-3-yl)amino)-2-fluorophenyl)piperidine-4-carbaldehyde (intermediate 2-3) instead of 1-(4-cyano-3-(trifluoromethyl)phenyl)piperidine-4-carboxylic acid (intermediate 1-1) and 1-(4-((2,6-dioxopiperidin-3-yl)amino)-2-fluorophenyl)piperidine-4-carbaldehyde (intermediate 2-1), respectively.

### Example 11. 1-(4-cyano-2-fluoro-3-(trifluoromethyl)phenyl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperidine-4-carboxamide

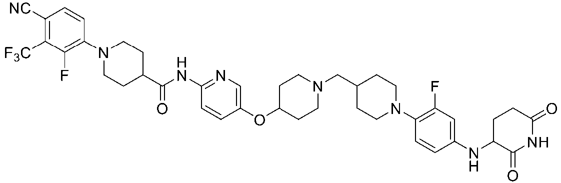

Example 11 was synthesized in the same manner as Example 1, using 1-(4-cyano-2-fluoro-3-(trifluoromethyl)phenyl)piperidine-4-carboxylic acid (intermediate 1-5) instead of 1-(4-cyano-3-(trifluoromethyl)phenyl)piperidine-4-carboxylic acid (intermediate 1-1).

### Example 12. 1-(3-chloro-4-cyano-2-fluorophenyl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperidine-4-carboxamide

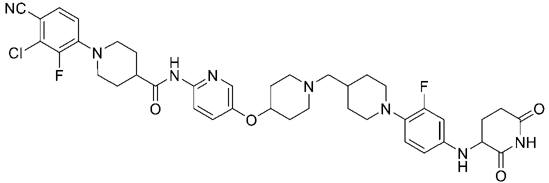

Example 12 was synthesized in the same manner as Example 1, using 1-(3-chloro-4-cyano-2-fluorophenyl)piperidine-4-carboxylic acid (intermediate 1-6) instead of 1-(4-cyano-3-(trifluoromethyl)phenyl)piperidine-4-carboxylic acid (intermediate 1-1).

### Example 13. 1-(4-cyano-3-methoxyphenyl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperidine-4-carboxamide

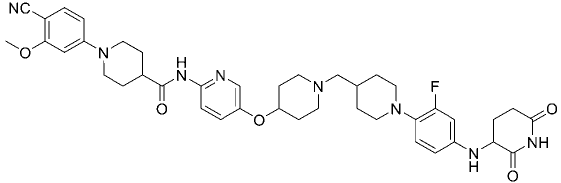

Example 13 was synthesized in the same manner as Example 1, using 1-(4-cyano-3-methoxyphenyl)piperidine-4-carboxylic acid (intermediate 1-7) instead of 1-(4-cyano-3-(trifluoromethyl)phenyl)piperidine-4-carboxylic acid (intermediate 1-1).

### Example 14. 1-(4-cyano-2-fluoro-3-(trifluoromethyl)phenyl)-N-(6-((1-((1-(4-((2,6-dioxopiperidin-3-yl)amino)-2-fluorophenyl)piperidin-4-yl)methyl)piperidin-4-yl)oxy)pyridazin-3-yl)piperidine-4-carboxamide

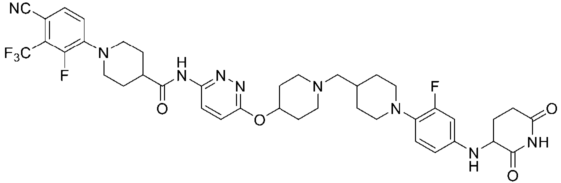

Example 14 was synthesized in the same manner as Example 7, using 1-(4-cyano-2-fluoro-3-(trifluoromethyl)phenyl)piperidine-4-carboxylic acid (intermediate 1-5) instead of 1-(4-cyano-3-(trifluoromethyl)phenyl)piperidine-4-carboxylic acid (intermediate 1-1).

### Example 15. 1-(3-chloro-4-cyano-2-fluorophenyl)-N-(6-((1-((1-(4-((2,6-dioxopiperidin-3-yl)amino)-2-fluorophenyl)piperidin-4-yl)methyl)piperidin-4-yl)oxy)pyridazin-3-yl)piperidine-4-carboxamide

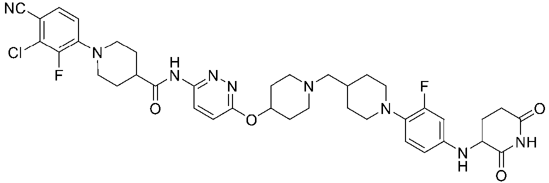

Example 15 was synthesized in the same manner as Example 7, using 1-(3-chloro-4-cyano-2-fluorophenyl)piperidine-4-carboxylic acid (intermediate 1-6) instead of 1-(4-cyano-3-(trifluoromethyl)phenyl)piperidine-4-carboxylic acid (intermediate 1-1).

### Example 16. 1-(4-cyano-3-methoxyphenyl)-N-(6-((1-((1-(4-((2,6-dioxopiperidin-3-yl)amino)-2-fluorophenyl)piperidin-4-yl)methyl)piperidin-4-yl)oxy)pyridazin-3-yl)piperidine-4-carboxamide

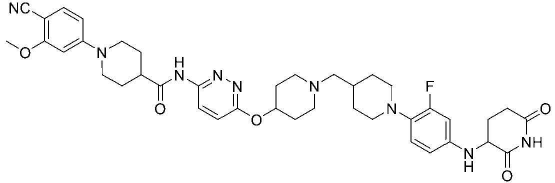

Example 16 was synthesized in the same manner as Example 7, using 1-(4-cyano-3-methoxyphenyl)piperidine-4-carboxylic acid (intermediate 1-7) instead of 1-(4-cyano-3-(trifluoromethyl)phenyl)piperidine-4-carboxylic acid (intermediate 1-1).

### Example 17. N-(5-((1-((1-(2-chloro-4-((2,6-dioxopiperidin-3-yl)amino)phenyl)piperidin-4-yl)methyl)piperidin-4-yl)oxy)pyridin-2-yl)-1-(4-cyano-3-(trifluoromethyl)phenyl)piperidine-4-carboxamide

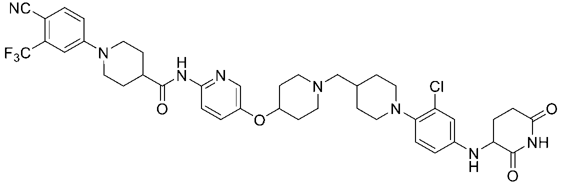

Example 17 was synthesized in the same manner as Example 1, using 1-(2-chloro-4-((2,6-dioxopiperidin-3-yl)amino)phenyl)piperidine-4-carbaldehyde (intermediate 2-5) instead of 1-(4-((2,6-dioxopiperidin-3-yl)amino)-2-fluorophenyl)piperidine-4-carbaldehyde (intermediate 2-1).

### Example 18. 1-(4-cyano-3-(trifluoromethyl)phenyl)-N-(5-((1-((1-(4-((2,6-dioxopiperidin-3-yl)amino)-2-(trifluoromethyl)phenyl)piperidin-4-yl)methyl)piperidin-4-yl)oxy)pyridin-2-yl)piperidine-4-carboxamide

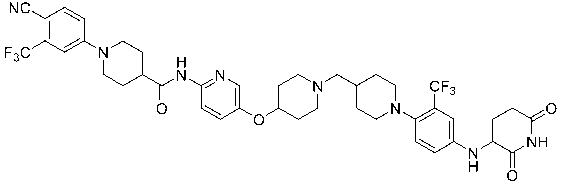

Example 18 was synthesized in the same manner as Example 1, using 1-(4-((2,6-dioxopiperidin-3-yl)amino)-2-(trifluoromethyl)phenyl)piperidine-4-carbaldehyde (intermediate 2-6) instead of 1-(4-((2,6-dioxopiperidin-3-yl)amino)-2-fluorophenyl)piperidine-4-carbaldehyde (intermediate 2-1).

The NMR and/or LC/MS results of the compounds synthesized above are summarized in the table below.

**[Table 1]**

| Example (Compound ) No. | NMR and/or MS data |
|---|---|
| 1 | ¹H NMR (400 MHz, DMSO-*d*₆) δ 10.78 (s, 1H), 10.37 (s, 1H), 8.02 (d, *J* = 2.9 Hz, 1H), 7.98 (d, *J* = 9.0 Hz, 1H), 7.81 (d, *J* = 8.8 Hz, 1H), 7.44 (dd, *J* = 9.1, 3.0 Hz, 1H), 7.33-7.29 (m, 1H), 7.28-7.23 (m, 1H), 6.82 (t, *J* = 9.2 Hz, 1H), 6.53-6.46 (m, 1H), 6.43-6.38 (m, 1H), 5.78 (d, *J* = 7.6 Hz, 1H), 4.42-4.32 (m, 1H), 4.30-4.20 (m, 1H), 4.15-4.06 (m, 2H), 3.14-3.07 (m, 2H), 3.04-2.95 (m, 2H), 2.80-2.62 (m, 7H), 2.21-2.14 (m, 4H), 2.12-2.04 (m, 1H), 1.96-1.90 (m, 2H), 1.88-1.81 (m, 3H), 1.80-1.71 (m, 2H), 1.70-1.62 (m, 2H), 1.62-1.50 (m, 3H), 1.28-1.18 (m, 2H). m/z 791.34 [M+H]⁺. |
| 2 | ¹H NMR (400 MHz, DMSO-*d*₆) δ 10.76 (s, 1H), 10.36 (s, 1H), 8.02 (d, *J* = 2.9 Hz, 1H), 7.98 (d, *J =* 9.1 Hz, 1H), 7.81 (d, *J* = 8.9 Hz, 1H), 7.44 (dd, *J* = 9.1, 2.9 Hz, 1H), 7.31 (d, *J* = 2.4 Hz, 1H), 7.25 (dd, *J* = 8.9, 2.4 Hz, 1H), 6.82 (t, *J* = 9.3 Hz, 1H), 6.49 (dd, *J* = 14.9, 2.4 Hz, 1H), 6.41 (dd, *J* = 8.7, 2.2 Hz, 1H), 5.77 (d, *J* = 7.6 Hz, 1H), 4.43-4.33 (m, 1H), 4.27-4.18 (m, 1H), 4.15-4.02 (m, 2H), 3.16-3.06 (m, 2H), 3.06-2.94 (m, 2H), 2.82-2.64 (m, 5H), 2.62-2.54 (m, 2H), 2.26-2.13 (m, 4H), 2.13-2.04 (m, 1H), 1.98-1.81 (m, 5H), 1.79-1.71 (m, 2H), 1.68-1.52 (m, 5H), 1.32-1.16 (m, 2H). m/z 791.44 [M+H]⁺. |
| 3 | ¹H NMR (400 MHz, DMSO-*d*₆) δ 10.79 (s, 1H), 10.39 (s, 1H), 8.02 (d, *J* = 2.9 Hz, 1H), 7.99 (d, *J* = 9.1 Hz, 1H), 7.82 (d, *J* = 8.9 Hz, 1H), 7.49-7.42 (m, 1H), 7.31 (d, *J* = 2.5 Hz, 1H), 7.29-7.21 (dd, *J* = 8.9, 2.5 Hz, 1H), 6.82 (t, *J* = 9.3 Hz, 1H), 6.53-6.45 (m, 1H), 6.44-6.37 (m, 1H), 5.79 (d, *J* = 7.6 Hz, 1H), 4.42-4.31 (m, 1H), 4.31-4.20 (m, 1H), 4.15-4.06 (m, 2H), 3.14-3.07 (m, 2H), 3.05-2.94 (m, 2H), 2.84-2.70 (m, 2H), 2.70-2.63 (m, 3H), 2.63-2.55 (m, 2H), 2.24-2.11 (m, 4H), 2.11-2.03 (m, 1H), 1.96-1.82 (m, 5H), 1.80-1.71 (m, 2H), 1.71-1.52 (m, 5H), 1.33-1.18 (m, 2H). m/z 791.45 [M+H]⁺. |
| 4 | ¹H NMR (400 MHz, DMSO-*d*₆) δ 10.79 (s, 1H), 10.39 (s, 1H), 8.02 (d, *J* = 2.9 Hz, 1H), 7.99 (d, *J* = 9.1 Hz, 1H), 7.82 (d, *J* = 8.9 Hz, 1H), 7.45 (dd, *J* = 9.1, 2.9 Hz, 1H), 7.31 (d, *J* = 2.0 Hz, 1H), 7.26 (dd, *J* = 8.9, 2.0 Hz, 1H), 6.83 (t, *J* = 9.3 Hz, 1H), 6.50 (dd, *J* = 15.0, 2.2 Hz, 1H), 6.40 (dd, *J* = 8.6, 1.6 Hz, 1H), 5.81 (d, *J* = 7.5 Hz, 1H), 4.41-4.31 (m, 1H), 4.31-4.20 (m, 1H), 4.17-4.06 (m, 2H), 3.19-3.11 (m, 2H), 3.05-2.95 (m, 2H), 2.86-2.77 (m, 2H), 2.77-2.70 (m, 1H), 2.69-2.64 (m, 1H), 2.59-2.54 (m, 2H), 2.44-2.29 (m, 4H), 2.12-2.04 (m, 1H), 1.98-1.90 (m, 2H), 1.90-1.81 (m, 3H), 1.81-1.74 (m, 2H), 1.69-1.55 (m, 6H). m/z 777.34 [M+H]⁺. |
| 5 | ¹H NMR (400 MHz, DMSO-*d*₆) δ 10.79 (s, 1H), 10.39 (s, 1H), 8.64 (d, *J =* 2.7 Hz, 1H), 8.02 (d, *J =* 3.0 Hz, 1H), 7.99 (d, *J* = 9.1 Hz, 1H), 7.63 (d, *J =* 2.7 Hz, 1H), 7.45 (dd, *J* = 9.1, 3.0 Hz, 1H), 6.82 (t, *J* = 9.3 Hz, 1H), 6.49 (dd, *J* = 15.0, 2.3 Hz, 1H), 6.41 (dd, *J* = 8.8, 2.3 Hz, 1H), 5.80 (d, *J* = 7.6 Hz, 1H), 4.42-4.33 (m, 1H), 4.29-4.18 (m, 3H), 3.15-3.02 (m, 4H), 2.86-2.76 (m, 1H), 2.76-2.62 (m, 4H), 2.60-2.53 (m, 2H), 2.22-2.12 (m, 4H), 2.12-2.03 (m, 1H), 1.96-1.87 (m, 4H), 1.87-1.80 (m, 1H), 1.79-1.71 (m, 2H), 1.70-1.64 (m, 2H), 1.63-1.55 (m, 3H), 1.27-1.18 (m, 2H). m/z 792.53 [M+H]⁺. |
| 6 | ¹H NMR (400 MHz, DMSO-*d*₆) δ 10.79 (s, 1H), 9.13 (s, 1H), 7.97 (d, *J* = 2.9 Hz, 1H), 7.86 (d, *J* = 8.8 Hz, 1H), 7.70 (d, *J* = 9.1 Hz, 1H), 7.41 (dd, *J* = 9.1, 2.9 Hz, 1H), 7.34 (d, 2.4 Hz, 1H), 7.26 (dd, *J* = 8.8, 2.4 Hz, 1H), 6.82 (t, *J = 9.3* Hz, 1H), 6.49 (d, *J* = 14.9, 2.4 Hz, 1H), 6.41 (d, *J* = 8.6, 1.6 Hz, 1H), 5.79 (d, *J* = 7.6 Hz, 1H), 4.40-4.29 (m, 1H), 4.29-4.20 (m, 1H), 3.64-3.59 (m, 4H), 3.55-3.50 (m, 4H), 3.15-3.06 (m, 2H), 2.78-2.71 (m, 1H), 2.71-2.62 (m, 3H), 2.61-2.54 (m, 2H), 2.22-2.14 (m, 4H), 2.12-2.04 (m, 1H), 1.97-1.88 (m, 2H), 1.87-1.80 (m, 1H), 1.79-1.71 (m, 2H), 1.68-1.56 (m, 3H), 1.29-1.17 (m, 2H). m/z 792.42 [M+H]⁺. |
| 7 | ¹H NMR (400 MHz, DMSO-*d*₆) δ 10.91 (s, 1H), 10.79 (s, 1H), 8.20 (d, *J* = 9.5 Hz, 1H), 7.82 (d, *J* = 8.9 Hz, 1H), 7.32 (d, *J* = 2.0 Hz, 1H), 7.26 (dd, *J* = 8.9, 2.0 Hz, 1H), 7.19 (d, *J* = 9.5 Hz, 1H), 6.83 (t, *J* = 9.4 Hz, 1H), 6.49 (dd, *J* = 15.0, 2.4 Hz, 1H), 6.41 (d, *J* = 8.7, 2.4 Hz, 1H), 5.79 (d, *J* = 7.7 Hz, 1H), 5.18-5.07 (m, 1H), 4.30-4.21 (m, 1H), 4.18-4.07 (m, 2H), 3.15-3.08 (m, 2H), 3.08-2.98 (m, 2H), 2.91-2.79 (m, 1H), 2.76-2.65 (m, 6H), 2.26-2.15 (m, 4H), 2.11-2.01 (m, 3H), 1.96-1.88 (m, 2H), 1.87-1.81 (m, 1H), 1.81-1.72 (m, 3H), 1.72-1.63 (m, 4H), 1.29-1.21 (m, 2H). m/z 792.51 [M+H]⁺. |
| 8 | ¹H NMR (400 MHz, DMSO-*d*₆) δ 10.79 (s, 1H), 10.38 (s, 1H), 8.02 (d, *J* = 3.0 Hz, 1H), 7.99 (d, *J* = 9.1 Hz, 1H), 7.64 (d, *J* = 9.0 Hz, 1H), 7.44 (dd, *J* = 9.0, 3.0 Hz, 1H), 7.17 (d, *J* = 2.4 Hz, 1H), 6.99 (dd, *J* = 9.1, 2.4 Hz, 1H), 6.82 (t, *J* = 9.3 Hz, 1H), 6.49 (dd, *J* = 15.0, 2.4 Hz, 1H), 6.41 (dd, *J* = 8.6, 2.4 Hz, 1H), 5.79 (d, *J* = 7.7 Hz, 1H), 4.43-4.32 (m, 1H), 4.29-4.20 (m, 1H), 4.10-3.99 (m, 2H), 3.16-3.05 (m, 2H), 3.01-2.92 (m, 2H), 2.82-2.71 (m, 2H), 2.71-2.63 (m, 3H), 2.61-2.54 (m, 2H), 2.24-2.12 (m, 4H), 2.12-2.04 (m, 1H), 1.97-1.88 (m, 2H), 1.88-1.79 (m, 3H), 1.79-1.70 (m, 2H), 1.69-1.52 (m, 5H), 1.28-1.20 (m, 2H). m/z 757.53 [M+H]⁺. |
| 9 | ¹H NMR (400 MHz, DMSO-*d*₆) δ 10.80 (s, 1H), 10.39 (s, 1H), 8.02 (d, *J =* 3.0 Hz, 1H), 7.98 (d, *J* = 9.1 Hz, 1H), 7.64 (d, *J* = 9.0 Hz, 1H), 7.44 (dd, *J* = 9.0, 3.0 Hz, 1H), 7.17 (d, *J* = 2.4 Hz, 1H), 6.99 (dd, *J* = 9.1, 2.4 Hz, 1H), 6.82 (t, *J* = 9.3 Hz, 1H), 6.49 (dd, *J* = 15.0, 2.4 Hz, 1H), 6.40 (dd, *J* = 8.6, 2.4 Hz, 1H), 5.80 (d, *J* = 7.7 Hz, 1H), 4.43-4.33 (m, 1H), 4.30-4.20 (m, 1H), 4.10-3.96 (m, 2H), 3.16-3.06 (m, 2H), 3.00-2.90 (m, 2H), 2.80-2.64 (m, 5H), 2.61-2.53 (m, 2H), 2.21-2.12 (m, 4H), 2.11-2.04 (m, 1H), 1.97-1.87 (m, 2H), 1.87-1.79 (m, 3H), 1.77-1.71 (m, 2H), 1.69-1.55 (m, 5H), 1.28-1.17 (m, 2H). m/z 757.49 [M+H]⁺. |
| 10 | ¹H NMR (400 MHz, DMSO-*d*₆) δ 10.77 (s, 1H), 10.36 (s, 1H), 8.02 (d, *J =* 3.0 Hz, 1H), 7.98 (d, *J* = 9.1 Hz, 1H), 7.63 (d, *J* = 9.0 Hz, 1H), 7.44 (dd, *J* = 9.0, 3.0 Hz, 1H), 7.16 (d, *J* = 2.4 Hz, 1H), 6.99 (dd, *J* = 9.1, 2.4 Hz, 1H), 6.82 (t, *J* = 9.3 Hz, 1H), 6.49 (dd, *J* = 15.0, 2.4 Hz, 1H), 6.41 (dd, *J* = 8.6, 2.4 Hz, 1H), 5.78 (d, *J* = 7.7 Hz, 1H), 4.42-4.31 (m, 1H), 4.28-4.21 (m, 1H), 4.10-3.99 (m, 2H), 3.16-3.05 (m, 2H), 3.01-2.92 (m, 2H), 2.83-2.70 (m, 2H), 2.70-2.63 (m, 3H), 2.59-2.54 (m, 2H), 2.22-2.13 (m, 4H), 2.13-2.04 (m, 1H), 1.97-1.88 (m, 2H), 1.88-1.80 (m, 3H), 1.80-1.71 (m, 2H), 1.67-1.53 (m, 5H), 1.31-1.16 (m, 2H). m/z 757.47 [M+H]⁺. |
| 11 | ¹H NMR (400 MHz, DMSO-*d*₆) δ 10.76 (s, 1H), 10.35 (s, 1H), 8.04-7.96 (m, 2H), 7.79 (d, *J* = 8.6 Hz, 1H), 7.48-7.42 (m, 2H), 6.87-6.78 (m, 1H), 6.49 (dd, *J* = 15.0, 2.4 Hz, 1H), 6.44-6.38 (m, 1H), 5.78-5.75 (m, 1H), 4.43-4.32 (m, 1H), 4.31-4.20 (m, 1H), 3.71-3.62 (m, 2H), 3.14-3.06 (m, 2H), 2.99-2.90 (m, 2H), 2.77-2.64 (m, 4H), 2.63-2.53 (m, 3H), 2.23-2.14 (m, 4H), 2.12-2.04 (m, 1H), 1.97-1.86 (m, 4H), 1.86-1.80 (m, 1H), 1.80-1.70 (m, 4H), 1.67-1.52 (m, 3H), 1.31-1.17 (m, 2H). m/z 809.70 [M+H]⁺. |
| 12 | ¹H NMR (400 MHz, DMSO-*d*₆) δ 10.76 (s, 1H), 10.35 (s, 1H), 8.10-7.95 (m, 2H), 7.73-7.60 (m, 1H), 7.45 (dd, *J* = 9.0, 2.9 Hz, 1H), 7.21-7.09 (m, 1H), 6.91-6.78 (m, 1H), 6.56-6.46 (m, 1H), 6.45-6.34 (m, 1H), 5.79-5.75 (m, 1H), 4.41-4.32 (m, 1H), 4.31-4.21 (m, 1H), 3.69-3.63 (m, 2H), 3.14-3.07 (m, 2H), 2.99-2.89 (m, 2H), 2.79-2.63 (m, 4H), 2.62-2.53 (m, 3H), 2.24-2.13 (m, 4H), 2.12-2.05 (m, 1H), 1.97-1.86 (m, 4H), 1.84-1.69 (m, 5H), 1.67-1.50 (m, 3H), 1.30-1.17 (m, 2H). m/z 775.71 [M+H]⁺. |
| 13 | ¹H NMR (400 MHz, CDCl₃) δ 8.16-8.07 (m, 2H), 7.96 (d, *J* = 2.9 Hz, 1H), 7.92 (s, 1H), 7.37 (d, *J* = 8.7 Hz, 1H), 7.31-7.27 (m, 1H), 6.91-6.84 (m, 1H), 6.46 (dd, *J* = 8.7, 2.1 Hz, 1H), 6.45-6.38 (m, 2H), 6.34 (d, *J* = 2.1 Hz, 1H), 4.58 (d, *J* = 3.6 Hz, 1H), 4.35-4.24 (m, 1H), 4.08-3.96 (m, 1H), 3.95-3.86 (m, 5H), 3.33-3.24 (m, 2H), 3.02-2.93 (m, 2H), 2.94-2.84 (m, 1H), 2.81-2.65 (m, 3H), 2.65-2.56 (m, 2H), 2.56-2.45 (m, 2H), 2.30-2.19 (m, 4H), 2.10-2.02 (m, 2H), 2.02-1.89 (m, 5H), 1.89-1.71 (m, 5H), 1.46-1.35 (m, 2H). m/z 753.74 [M+H]⁺. |
| 14 | ¹H NMR (400 MHz, CDCl₃) δ 8.41-8.33 (m, 2H), 7.89 (s, 1H), 7.50 (d, *J* = 8.6 Hz, 1H), 7.13-7.07 (m, 1H), 7.00 (d, *J* = 9.6 Hz, 1H), 6.92-6.85 (m, 1H), 6.46-6.37 (m, 2H), 5.29-5.17 (m, 1H), 4.58 (d, *J* = 3.6 Hz, 1H), 4.04-3.95 (m, 1H), 3.77-3.66 (m, 2H), 3.35-3.24 (m, 2H), 3.05-2.95 (m, 2H), 2.92-2.84 (m, 1H), 2.81-2.69 (m, 3H), 2.64-2.56 (m, 3H), 2.56-2.50 (m, 1H), 2.31-2.22 (m, 4H), 2.16-2.05 (m, 6H), 2.05-1.97 (m, 1H), 1.97-1.88 (m, 1H), 1.88-1.79 (m, 4H), 1.49-1.35 (m, 2H). m/z 810.71 [M+H]⁺. |
| 15 | ¹H NMR (400 MHz, CDCl₃) δ 8.47 (s, 1H), 8.38 (d, *J* = 9.5 Hz, 1H), 7.94 (s, 1H), 7.36 (dd, *J* = 8.6, 1.5 Hz, 1H), 7.00 (d, *J* = 9.5 Hz, 1H), 6.92-6.80 (m, 2H), 6.45-6.38 (m, 2H), 5.27-5.18 (m, 1H), 4.58 (d, *J* = 3.6 Hz, 1H), 4.04-3.95 (m, 1H), 3.77-3.65 (m, 2H), 3.36-3.22 (m, 2H), 3.02-2.93 (m, 2H), 2.92-2.84 (m, 1H), 2.80-2.69 (m, 3H), 2.63-2.50 (m, 4H), 2.33-2.22 (m, 4H), 2.15-2.04 (m, 6H), 2.03-1.92 (m, 1 H), 1.92-1.78 (m, 5H), 1.49-1.34 (m, 2H). m/z 776.71 [M+H]⁺. |
| 16 | ¹H NMR (400 MHz, CDCl₃) δ 8.66 (s, 1H), 8.38 (d, *J* = 9.5 Hz, 1H), 8.00 (s, 1H), 7.38 (d, *J* = 8.7 Hz, 1H), 7.00 (d, *J* = 9.5 Hz, 1H), 6.93-6.85 (m, 1H), 6.47 (dd, *J* = 8.7, 2.1 Hz, 1H), 6.45-6.38 (m, 2H), 6.34 (d, *J* = 2.1 Hz, 1H), 5.27-5.16 (m, 1H), 4.59 (d, *J* = 3.6 Hz, 1H), 4.03-3.96 (m, 1H), 3.95-3.87 (m, 5H), 3.34-3.25 (m, 2H), 3.06-2.96 (m, 2H), 2.91-2.83 (m, 1H), 2.83-2.69 (m, 3H), 2.69-2.49 (m, 4H), 2.31-2.21 (m, 4H), 2.15-2.04 (m, 4H), 2.02-1.90 (m, 3H), 1.89-1.78 (m, 5H), 1.47-1.35 (m, 2H). m/z 754.74 [M+H]⁺. |
| 17 | ¹H NMR (400 MHz, CDCl₃) δ 8.45 (s, 1H), 8.13 (d, *J =* 9.0 Hz, 1H), 8.04 (s, 1H), 7.98 (d, *J* = 2.8 Hz, 1H), 7.62 (d, *J* = 8.8 Hz, 1H), 7.30-7.27 (m, 1H), 7.14 (d, *J =* 2.3 Hz, 1H), 6.98 (dd, *J* = 8.8, 2.3 Hz, 1H), 6.95 (d, *J =* 8.7 Hz, 1H), 6.72 (d, *J =* 2.7 Hz, 1H), 6.57 (dd, *J* = 8.7, 2.7 Hz, 1H), 4.56 (d, *J* = 3.9 Hz, 1H), 4.33-4.24 (m, 1H), 4.06-3.93 (m, 3H), 3.31-3.22 (m, 2H), 3.14-3.03 (m, 2H), 2.92-2.84 (m, 1H), 2.82-2.68 (m, 3H), 2.65-2.48 (m, 4H), 2.30-2.19 (m, 4H), 2.13-2.04 (m, 2H), 2.03-1.90 (m, 5H), 1.90-1.72 (m, 5H), 1.47-1.35 (m, 2H). m/z 807.66 [M+H]⁺. |
| 18 | ¹H NMR (400 MHz, CDCl₃) δ 8.36 (s, 1H), 8.12 (d, *J* = 9.1 Hz, 1H), 8.07 (s, 1H), 7.96 (d, *J* = 2.8 Hz, 1H), 7.61 (d, *J* = 8.8 Hz, 1H), 7.29-7.26 (m, 1H), 7.23 (d, *J* = 8.8 Hz, 1H), 7.13 (d, *J =* 2.4 Hz, 1H), 6.97 (dd, *J* = 8.7, 2.7 Hz, 1H), 6.86 (d, *J* = 2.7 Hz, 1H), 6.79 (dd, *J* = 8.7, 2.7 Hz, 1H), 4.70 (d, *J* = 4.1 Hz, 1H), 4.32-4.21 (m, 1H), 4.10-4.01 (m, 1H), 4.01-3.92 (m, 2H), 3.11-3.02 (m, 2H), 2.98-2.91 (m, 2H), 2.91-2.84 (m, 1H), 2.82-2.75 (m, 1H), 2.75-2.67 (m, 2H), 2.66-2.58 (m, 2H), 2.58-2.48 (m, 2H), 2.27-2.18 (m, 4H), 2.10-2.03 (m, 2H), 2.02-1.89 (m, 5H), 1.89-1.71 (m, 5H), 1.41-1.28 (m, 2H). m/z 841.76 [M+H]⁺. |

### Experimental Example 1: Measurement of Androgen Receptor (AR) Protein Degradation

The amount of AR protein present in LNCaP or VCaP cells was measured after treating them with the synthesized compound. The amount of AR protein was measured using Western blotting. The protocol for the experiments using LNCaP or VCaP cells was as follows.

[Culture] LNCaP cells were resuspended in RPMI1640 medium + HEPES (Hyclone, SH30255.01), 10% FBS (Hyclone, SV30207.02), and 1% Penicillin-streptomycin (Welgene, LS 202-02). 3 x 10⁵ cells/mL were seeded in 12-well plates (1 mL per well) and cultured for 3 days. VCaP cells were resuspended in IMDM MEDIUM (Hyclone, SH30228.01), 20% FBS (Hyclone, SV30207.02), and 1% Penicillin-streptomycin (Welgene, LS 202-02), seeded at 9 x 10⁵/mL in 1 mL per 12-well plate, and cultured for 3 days.

[Compound Treatment] 10 µL of 100x stock (e.g., 0.1, 1, 10 µM) compound solution at the final compound treatment concentration (e.g., 0.001, 0.01, 0.1 µM) was added to each well in 1 mL media, and cells were harvested after 20 hours. For DC₅₀ measurement, 100x stock (0.01, 0.1, 1, 10, 100, 1000 µM) compound solutions at the final compound treatment concentration (0.0001, 0.001, 0.01, 0.1, 1, 10 µM) were used.

[Cell lysis] The cell pellet was suspended using 1% SDS lysis buffer (50 mM Tris, 1 mM EDTA, 1% SDS, pH 8.0, 0.5 mM PMSF, 1x Protease/Phosphatase inhibitor Cocktail (Cell Signaling Technology, 5872S)), and sonication (70% AMP, 10 cycles (30 sec ON/59 sec OFF)) was performed. After centrifugation at 4°C and 20,000 g for 20 minutes, the cell lysate was obtained and transferred to a new tube.

[Protein Quantification] A BCA protein assay (SMART^{™} BCA Protein Assay Kit, iNtRON Biotechnology, 21071) was performed using a 96-well microplate. 5 µL of cell lysate and 5 µL of 1% SDS lysis buffer were added to each well of the 96-well plate, followed by an additional 200 µL of BCA reagent (Reagent A: Reagent B = 50:1). The wells were incubated at 37°C for 30 minutes and cooled to room temperature for 10 minutes. After shaking for 10 seconds, the absorbance was measured at a wavelength of 562 nm. The unknown protein concentration was determined using a BSA standard curve (0, 0.125, 0.25, 0.5, 1, 2 mg/mL). Western blotting samples were prepared by mixing cell lysate, 1% SDS lysis buffer, 4x NuPAGE^{™} LDS Sample buffer (Invitrogen, NP0008), and 10X NuPAGE^{™} Sample Reducing Agent (Invitrogen, NP0009) and boiling at 70°C for 10 minutes.

[Electrophoresis] 10 µg of protein sample was loaded into each well of a NuPAGE^{™} 4 to 12%, Bis-Tris, 1.0 mm Mini Protein Gel, 15-well, 17-well, or 26-well (Invitrogen, NP0323BOX, NP0329BOX, or WG1403BOX) and electrophoresed for 40 minutes using NuPAGE^{™}MES SDS Running Buffer (Invitrogen, NP0002) at 200 V (constant V).

[Transfer] Transfer was performed for 15 or 18 minutes using the electrophoresed gel and the Trans-Blot^{®} Turbo^{™} Transfer Starter System (Bio-RAD, #17001918, Mini Nitrocellulose) at 1.3 A (constant A) and 25 V (limit V). To confirm protein transfer, 15 mL of Ponceau S solution (Sigma, P7170-1L) was used to stain for 1 minute, and then washed to check the degree of transfer.

[Blocking] The membrane for AR and GAPDH detection was blocked with 5% skim milk/0.2% TBST (Skim milk: BD, 232100/ 20X TBS: Biosesang, TR2008-100-00/ 10% Tween 20 Solution: Bio-RAD, #161-0781) for 45 minutes.

[Antibodies and Detection] Primary AR antibody (Androgen Receptor: CST, 5153S) was mixed with 5% skim milk/0.2% TBST at a ratio of 1:1,000 and reacted at room temperature for 3 hours or at 4°C overnight. Primary GAPDH antibody (CST, 2118S) was mixed with 5% skim milk/0.2% TBST at a ratio of 1:2,000 and reacted at room temperature for 3 hours or at 4°C overnight. Afterwards, TBST (0.2% Tween 20) was used for washing three times every 5 minutes. Secondary antibody to AR antibody, Anti-rabbit IgG, HRP-linked Antibody (CST, 7074S), was mixed with 5% skim milk/0.2% TBST at a ratio of 1:5,000 and reacted at room temperature for 45 minutes. Secondary antibody against GAPDH antibody, IRDye^{®} 800CW Goat anti-Rabbit IgG Secondary Antibody, was mixed at a ratio of 1:10,000 in 5% skim milk/0.2% TBST and incubated at room temperature for 45 minutes. After 45 minutes, the membrane was washed five times with TBST (0.2% Tween 20) every 5 minutes. After reacting the membrane with SuperSignal^{™} West Pico Plus Chemiluminescent Substrate (Thermo Scientific, 34580), detection was performed using the Odyssey^{®} Fc Imaging system (LI-COR, 2800).

[Quantification of Numerical Values] The amount of AR protein was measured using a program embedded in the Odyssey^{®} Fc Imaging system (LI-COR, 2800). The measured amount of GAPDH protein was normalized to yield % degradation, Dₘₐₓ, and DC₅₀.

The results are presented in the tables below.

**[Table 2]**

| Example No. | AR degradation in LNCaP cells (%) | |
|---|---|---|
| | 10 nM | 100 nM |
| 1 | 84 | 99 |
| 2 | 86 | 99 |
| 3 | 93 | 99 |
| 4 | 76 | 93 |
| 5 | 63 | 97 |
| 6 | 84 | 99 |
| 7 | 79 | 93 |
| 8 | 91 | 98 |
| 9 | 86 | 96 |
| 10 | 90 | 99 |
| 12 | 88 | 96 |
| 13 | 91 | 99 |
| 15 | 88 | 90 |
| 16 | 93 | 98 |
| 17 | 77 | 99 |
| 18 | 51 | 98 |

**[Table 3]**

| Example No. | AR degradation in VCaP cells (%) | |
|---|---|---|
| | 10 nM | 100 nM |
| 1 | 89 | 99 |
| 2 | 83 | 99 |
| 3 | 72 | 96 |
| 7 | 94 | 100 |
| 8 | 94 | 99 |
| 9 | 94 | 99 |
| 10 | 85 | 99 |
| 11 | 89 | 99 |
| 12 | 94 | 98 |
| 13 | 95 | 100 |
| 14 | 87 | 95 |
| 15 | 98 | 100 |
| 16 | 99 | 100 |
| 17 | 75 | 97 |

**[Table 4]**

| Example No. | AR degradation in VCaP cells | |
|---|---|---|
| | DC₅₀ (nM) | Dₘₐₓ (%) |
| 2 | 0.19 | 99 |
| 9 | 0.10 | 99 |

As shown in the table above, the compounds of the present invention exhibited excellent activity in degrading the androgen receptor.

### Experimental Example 2: Pharmacodynamic Efficacy Measurement

The protocol for the creation of a xenograft model and pharmacodynamic experiments using VCaP cells was as follows.

[Cell Culture] VCaP cells were resuspended at a density of 4 x 10⁵/mL in a mixture of DMEM MEDIUM (Hyclone, SH30243.01), 10% FBS (Hyclone, SV30207.02), 1% Penicillin-streptomycin (Welgene, LS 202-02), and 1x Non-essential amino acid solution (100x) (Sigma, M7145-100mL). 50 mL of the cells were seeded into CELLBIND cell culture flasks (150 cm²) (Corning, 3291) and cultured for 5 days.

[Xenotransplantation] The media was removed from the flask containing cultured VCaP cells and washed with 10 mL of PBS (Welgene, LB001-02). After removing the PBS, 2 mL of 1x 0.05% Trypsin-EDTA (Welgene, LS015-01) was added and treated in a cell incubator for 3 minutes. Then, 10 mL of media was added, pipetted, and placed in a 50 mL tube. The pellets were collected by centrifugation at 500 g for 5 minutes. The collected pellets were washed once more with 20 mL of PBS. The washed pellets were collected by centrifugation at 500 g for 5 minutes. The PBS was removed, and 500 µL of HBSS (Gibco, 14025092) was added and pipetted. After diluting 100-fold in HBSS and measuring the cell count, the concentration was calculated as 3 x 10⁶/50 µL/mouse, diluted in HBSS, and stored in an ice bucket. An equal volume of matrigel (R&D system, 3432-005-01) was added to the stored cells, homogenized by pipetting, and then 100 µL per mouse was inoculated subcutaneously into the right flank of CB17/SCID mice.

[Measurement of tumor size and division into groups] After 2-3 weeks of inoculation with cancer cells, the transplanted cancer cells grew and were measured in size using a caliper. The measured tumor size was calculated as [long axis x short axis² x 0.5]. When the tumor size exceeded approximately 200 mm², the experimental animals were divided into groups based on the average tumor size and the concentration of the drug to be administered. The groups always included a group containing only the solvent (vehicle) without the drug.

[Drug Preparation and Administration] The drug was dissolved in a solvent and orally administered to each experimental animal in a volume of 200 µL (approximately 8 mL/kg). The solvent composition was 3% DMSO (Sigma, D8418), 10% Cremophor EL (Sigma, C5135), 20% PEG400 (Sigma, 06855-1KG), and 67% distilled water. The preparation method was as follows. Depending on the drug concentration (e.g., 10 or 30 mg/kg), each drug was weighed and dissolved in DMSO corresponding to 3% of the total solvent volume. 10% Cremophor EL and 20% PEG400 were added to the drug dissolved in DMSO, vortexed, and finally 67% DW was added and vortexed again. The prepared drug was orally administered according to the experimental animal group.

[Cancer Tissue Extraction] The prepared drug was administered orally once daily at the same time for three days. Changes in tumor size were measured during the prescribed administration period, and the mice were sacrificed 6 hours after the final administration. After sacrifice, tumor tissue was collected, washed in PBS, and stored at -80°C.

[Tissue homogenization and cell lysis] The cancer tissue stored at -80°C was thawed on ice and cut into equal sizes of 0.5 cm x 0.5 cm x 0.5 cm (length x width x height). One 2.8 mm ceramic bead (Omni international Inc., 19-646-3) and seven 1.4 mm ceramic beads (Omni international Inc., 19-645-3) were added to an E.tube. The cut cancer tissue and 1% SDS lysis buffer (50 mM Tris, 1 mM EDTA, 1% SDS, pH 8.0, 0.5 mM PMSF, 1x Protease/Phosphatase inhibitor Cocktail (Cell Signaling Technology, 5872S)) were added thereto, and homogenized using a bead mill homogenizer (Omni international Inc., #19-040E). Afterwards, sonication (70% AMP, 10 cycles (10 sec ON/30 sec OFF)) was performed, and after centrifugation at 4°C, 20,000g for 15 minutes, cell lysate was obtained as the supernatant.

[Western blotting sample preparation] Western blotting samples were prepared by mixing cell lysate, 1% SDS lysis buffer, 4x NuPAGE^{™} LDS Sample buffer (Invitrogen, NP0008), and 10x NuPAGE^{™} Sample Reducing Agent (Invitrogen, NP0009) and boiling the mixture at 70°C for 10 minutes.

[Electrophoresis] 10 µL of the prepared sample was loaded into each well of NuPAGE^{™} 4 to 12%, Bis-Tris, 1.0 mm Mini Protein Gel, 15-well or 17-well (Invitrogen, NP0323BOX or NP0329BOX). Electrophoresis was then performed for 40 minutes at 200 V (constant V) using NuPAGE^{™} MES SDS Running Buffer (Invitrogen, NP0002).

[Transfer] Transfer was performed for 15 minutes using the electrophoresis-performed gel and the Trans-Blot^{®} Turbo^{™} Transfer Starter System (Bio-RAD, #17001918, Mini Nitrocellulose) at 1.3 A (Constant A) and 25 V (Limit V). To confirm protein transfer, 15 mL of Ponceau S solution (Sigma, P7170-1L) was used for staining for 1 minute, and the degree of transfer was confirmed by washing.

[Blocking] The membrane for AR and GAPDH detection was blocked with 5% skim milk/0.2% TBST (Skim milk: BD, 232100 / 20x TBS: Biosesang, TR2008-100-00 / 10% Tween 20 Solution: Bio-RAD, #161-0781) for 45 minutes.

[Antibodies and Detection] The primary AR antibody (Androgen Receptor: CST, 5153S) was mixed with 5% skim milk/0.2% TBST at a ratio of 1:1,000 and reacted overnight at 4°C. The primary GAPDH antibody (CST, 2118S) was mixed with 5% skim milk/0.2% TBST at a ratio of 1:1,000 and reacted overnight at 4°C. Afterwards, the plate was washed three times every 5 minutes with TBST (0.2% Tween 20). The secondary antibody, Anti-rabbit IgG, HRP-linked Antibody (CST, 7074S) against AR and GAPDH antibodies was mixed with 5% skim milk/0.2% TBST at a ratio of 1:5,000 and reacted at room temperature for 45 minutes. After 45 minutes, the membrane was washed five times with TBST (0.2% Tween 20) every 5 minutes. After reacting with the membrane using SuperSignal^{™} West Pico Plus Chemiluminescent Substrate (Thermo Scientific, 34580), detection was performed using the Odyssey^{®} Fc Imaging system (LI-COR, 2800).

[Quantification of Numerical Values] The amount of AR protein was measured using a program embedded in the Odyssey^{®} Fc Imaging system (LI-COR, 2800). The measured amount was normalized to the amount of GAPDH protein, and AR % degradation was calculated.

The results of this pharmacodynamic evaluation are presented in the table below.

**[Table 5]**

| Example No. | Dosage | AR degradation in tumor (%) |
|---|---|---|
| 1 | 10 mg/kg | 86 |
| 2 | 10 mg/kg | 96 |
| 3 | 10 mg/kg | 89 |
| 9 | 10 mg/kg | 97 |

As shown in the table above, the compounds of the present invention exhibited excellent AR degradation activity after oral administration, demonstrating excellent pharmacodynamic properties.

### Experimental Example 3: Analysis of Plasma and Tumor Tissue Concentrations Following Oral Administration

The plasma and tumor tissue concentrations of the drug administered orally to a xenograft model using VCaP cells were analyzed as follows.

[Blood collection and tumor tissue extraction] The prepared drug was administered orally at a dose of 10 mg/kg once daily at the same time for 3 days. The mice were sacrificed 2, 6, and 24 hours after the last administration. Blood collection and tumor tissue extraction were performed immediately after sacrifice at each hour. Blood collected from the heart was placed in a K2EDTA tube for 30 minutes and then centrifuged at 15,000 rpm at 4°C for 10 minutes. The supernatant plasma was separated and stored at -20°C for analysis. The extracted tumor tissue was washed in PBS, cut into pieces of approximately 10 mg, and stored at -80°C for analysis.

[Sample Preparation] Cancer tissue was homogenized once at 5,500 rpm for 20 seconds after adding distilled water 9 times the tissue weight using a tissue homogenizer (Precellys Evolution, Bertin Technology). For cancer tissue, 20 µL of the homogenate was mixed with 20 µL of blank plasma sample, 20 µL of internal standard (carbamazepine, 30 ng/mL in acetonitrile), and 140 µL of acetonitrile. For plasma, 20 µL of the plasma sample was mixed with 20 µL of blank liver tissue sample, 20 µL of internal standard, and 140 µL of acetonitrile. Afterwards, the mixed sample was suspended for 10 minutes using a vortex mixer and centrifuged at 13,500 rpm for 10 minutes at room temperature. 140 µL of the supernatant was collected and transferred to an analysis container.

[Sample Analysis] Drug concentrations in cancer tissue and plasma samples were quantified using an HPLC/MS/MS system. 5 µL of the sample prepared in the analytical vessel was injected into the HPLC/MS/MS system for analysis. For drug quantification, a combination of 0.1% formic acid in distilled water and 0.1% formic acid in acetonitrile was used as the mobile phase. The analytical column used was a reverse-phase C18 column, and chromatograms were analyzed using isocratic elution. Ion detection was performed in ESI positive MRM mode, and peak integration was performed using Analyst software version 1.6.4 (Applied Biosystems/MDS SCIEX). Drug concentrations in cancer tissue were calculated by multiplying the concentrations obtained from the analysis of cancer tissue homogenate by a dilution factor of 10.

The results of the drug concentration analysis in plasma and cancer tissue are presented in the table below.

**[Table 6]**

| Example No. | Time | Concentration in Plasma (ng/mL) | Concentration in Tumor Tissue (ng/g) | Tissue to Plasma Ratio |
|---|---|---|---|---|
| 2 | 2 | 816 | 5563 | 6.78 |
| | 6 | 610 | 6127 | 10.10 |
| | 24 | 51 | 834 | 16.29 |

As shown in the above table, it was confirmed that the compound according to the present invention has significantly superior pharmacodynamic properties by showing an excellent tumor tissue to plasma concentration ratio after oral administration.

## Claims

1. A compound of Chemical Formula 1: 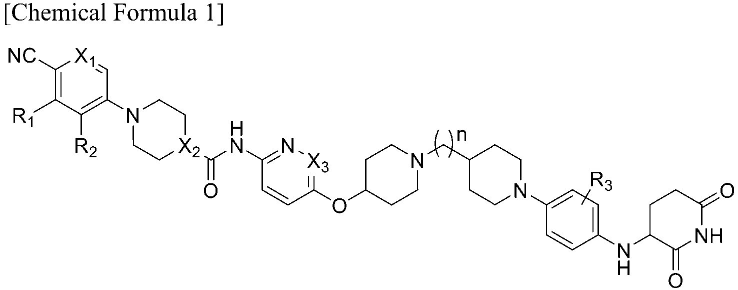
or a pharmaceutically acceptable salt thereof,
in the Chemical Formula 1,
R₁ is H, halogen, C₁₋₃alkyl, C₁₋₃alkoxy, or haloC₁₋₃alkyl,
R₂ is H, halogen, or haloC₁₋₃alkyl,
X₁, X₂, and X₃ are each independently CH or N,
R₃ is H, halogen, C₁₋₃alkyl, or haloC₁₋₃alkyl, and
n is 0, 1, or 2.

2. The compound of Claim 1 or a pharmaceutically acceptable salt thereof, in the Chemical Formula 1,
R₁ is H, Cl, -CH₃, -OCH₃, or -CF₃,
R₂ is H or F,
X₁, X₂, and X₃ are each independently CH or N,
R₃ is H, F, Cl, -CH₃, or -CF₃, and
n is 0, 1, or 2.

3. The compound of Claim 2 or a pharmaceutically acceptable salt thereof, in the Chemical Formula 1,
R₁ is Cl, -OCH₃, or -CF₃,
R₂ is H or F,
X₁, X₂, and X₃ are each independently CH or N,
R₃ is F, Cl, or -CF₃, and
n is 0 or 1.

4. The compound of Claim 3 or a pharmaceutically acceptable salt thereof, in the Chemical Formula 1,
R₁ is Cl, -OCH₃, or -CF₃,
R₂ is H or F,
X₁ and X₂ are CH and X₃ is CH or N,
R₃ is F or Cl, and
n is 1.

5. The compound of Claim 4 or a pharmaceutically acceptable salt thereof, in the Chemical Formula 1, 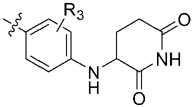 is

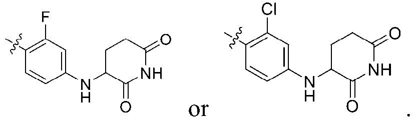

6. The compound of Claim 1 or a pharmaceutically acceptable salt thereof, wherein the compound is
1-(4-cyano-3-(trifluoromethyl)phenyl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperidine-4-carboxamide,
(S)-1-(4-cyano-3-(trifluoromethyl)phenyl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperidine-4-carboxamide,
(R)-1-(4-cyano-3-(trifluoromethyl)phenyl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperidine-4-carboxamide,
1-(4-cyano-3-(trifluoromethyl)phenyl)-N-(5-((1'-(4-((2,6-dioxopiperidin-3-yl)amino)-2-fluorophenyl)-[1,4'-bipiperidin]-4-yl)oxy)pyridin-2-yl)piperidine-4-carboxamide,
1-(6-cyano-5-(trifluoromethyl)pyridin-3-yl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperidine-4-carboxamide,
4-(4-cyano-3-(trifluoromethyl)phenyl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperazine-1-carboxamide,
1-(4-cyano-3-(trifluoromethyl)phenyl)-N-(6-((1-((1-(4-((2,6-dioxopiperidin-3-yl)amino)-2-fluorophenyl)piperidin-4-yl)methyl)piperidin-4-yl)oxy)pyridazin-3-yl)piperidine-4-carboxamide,
1-(3-chloro-4-cyanophenyl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperidine-4-carboxamide,
(S)-1-(3-chloro-4-cyanophenyl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperidine-4-carboxamide,
(R)-1-(3-chloro-4-cyanophenyl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperidine-4-carboxamide,
1-(4-cyano-2-fluoro-3-(trifluoromethyl)phenyl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperidine-4-carboxamide,
1-(3-chloro-4-cyano-2-fluorophenyl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperidine-4-carboxamide,
1-(4-cyano-3-methoxyphenyl)-N-(5-((1-((1-(4-((2,6-dioxopiperidin-3-yl)amino)-2-fluorophenyl)piperidin-4-yl)methyl)piperidin-4-yl)oxy)pyridin-2-yl)piperidine-4-carboxamide,
1-(4-cyano-2-fluoro-3-(trifluoromethyl)phenyl)-N-(6-((1-((1-(4-((2,6-dioxopiperidin-3-yl)amino)-2-fluorophenyl)piperidin-4-yl)methyl)piperidin-4-yl)oxy)pyridazin-3-yl)piperidine-4-carboxamide,
1-(3-chloro-4-cyano-2-fluorophenyl)-N-(6-((1-((1-(4-((2,6-dioxopiperidin-3-yl)amino)-2-fluorophenyl)piperidin-4-yl)methyl)piperidin-4-yl)oxy)pyridazin-3-yl)piperidine-4-carboxamide,
1-(4-cyano-3-methoxyphenyl)-N-(6-((1-((1-(4-((2,6-dioxopiperidin-3-yl)amino)-2-fluorophenyl)piperidin-4-yl)methyl)piperidin-4-yl)oxy)pyridazin-3-yl)piperidine-4-carboxamide,
N-(5-((1-((1-(2-chloro-4-((2,6-dioxopiperidin-3-yl)amino)phenyl)piperidin-4-yl)methyl)piperidin-4-yl)oxy)pyridin-2-yl)-1-(4-cyano-3-(trifluoromethyl)phenyl)piperidine-4-carboxamide, or
1-(4-cyano-3-(trifluoromethyl)phenyl)-N-(5-((1-((1-(4-((2,6-dioxopiperidin-3-yl)amino)-2-(trifluoromethyl)phenyl)piperidin-4-yl)methyl)piperidin-4-yl)oxy)pyridin-2-yl)piperidine-4-carboxamide.

7. A composition comprising the compound of any one of claims 1 to 6, or a pharmaceutically acceptable salt thereof, and a pharmaceutically acceptable carrier.

8. A pharmaceutical composition for inhibiting or degrading the androgen receptor (AR), comprising the compound of any one of claims 1 to 6, or a pharmaceutically acceptable salt thereof, as an active ingredient.

9. The pharmaceutical composition of claim 8, wherein the pharmaceutical composition is for the treatment or prevention of cancer, Kennedy's disease, acne, alopecia, cutaneous wounds, or hirsutism.

10. The pharmaceutical composition of claim 9, wherein the pharmaceutical composition is for the treatment or prevention of prostate cancer or breast cancer.

11. A method for treating or preventing an androgen receptor-related disease, comprising administering to a subject in need thereof a therapeutically effective amount of a compound of any one of claims 1 to 6, or a pharmaceutically acceptable salt thereof.

12. The method of claim 11, wherein the androgen receptor-related disease is cancer, Kennedy's disease, acne, alopecia, cutaneous wounds, or hirsutism.

13. The method of claim 12, wherein the androgen receptor-related disease is prostate cancer or breast cancer.
